(19) Europäisches Patentamt
European Patent Office
Office européen des brevets

(11) **EP 4 767 939 A1**

(12) **EUROPEAN PATENT APPLICATION**
published in accordance with Art. 153(4) EPC

(43) Date of publication:
**01.07.2026 Bulletin 2026/27**

(21) Application number: **25802776.2**

(22) Date of filing: **07.05.2025**

(51) International Patent Classification (IPC):
**A61B 5/1455** (2006.01)　　**A61B 5/0205** (2006.01)
**A61B 5/00** (2006.01)

(86) International application number:
**PCT/CN2025/093067**

(87) International publication number:
**WO 2025/237125 (20.11.2025 Gazette 2025/47)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH LA MA MD TN**

(30) Priority: **11.05.2024　CN 202410600826**

(71) Applicant: **HUAWEI TECHNOLOGIES CO., LTD.
Shenzhen, Guangdong 518129 (CN)**

(72) Inventors:
• **LI, Yue
  Guangdong 518129 (CN)**
• **QIU, Chunkai
  Guangdong 518129 (CN)**
• **XIA, Kailun
  Guangdong 518129 (CN)**
• **WEI, Xiaosong
  Guangdong 518129 (CN)**

(74) Representative: **Goddar, Heinz J.
Boehmert & Boehmert
Anwaltspartnerschaft mbB
Pettenkoferstrasse 22
80336 München (DE)**

(54) **METHOD AND RELATED APPARATUS FOR PHYSIOLOGICAL PARAMETER MEASUREMENT**

(57) This application discloses a physiological parameter detection method and a related apparatus. The method is applied to an electronic device including a PPG assembly. The PPG assembly may include a plurality of detection modules, and each detection module includes one light source and one photoelectric detector. The method may include: collecting a plurality of PPG signals via the plurality of detection modules, where each PPG signal is obtained in a manner in which a light source in one detection module emits a light ray toward skin and a photoelectric detector in the one detection module detects a light ray reflected from the skin, the plurality of PPG signals include at least signals respectively collected by two detection modules with different spacings, and a spacing of a detection module is a distance between a light source and a photoelectric detector in the detection module. Then, a physiological parameter value related to an arterial activity of a user may be determined based on the plurality of PPG signals. In this way, a physiological parameter can be measured more accurately by using the plurality of PPG signals.

| |
|---|
| Collect n PPG signals via n detection modules in a PPG assembly, where the n PPG signals include at least signals respectively collected by two detection modules with different spacings, and a spacing of a detection module is a distance between a light source and a photoelectric detector in the detection module ～ S101 |
| ↓ |
| Determine a physiological parameter value of a user based on the n PPG signals, where the physiological parameter value is related to an arterial activity of the user ～ S102 |

**FIG. 4**

## Description

This application claims priority to Chinese Patent Application No. 202410600826.5, filed with the China National Intellectual Property Administration on May 11, 2024 and entitled "PHYSIOLOGICAL PARAMETER DETECTION METHOD AND RELATED APPARATUS", which is incorporated herein by reference in its entirety.

## TECHNICAL FIELD

This application relates to the field of terminal and computer technologies, and in particular, to a physiological parameter detection method and a related apparatus.

## BACKGROUND

With continuous progress of science and technology, more devices are designed to monitor a physiological parameter of a user, including blood oxygen, blood glucose, heart rate, body temperature, blood pressure, blood lipid, blood ketone, respiratory rate, or the like.

Photoplethysmography (photoplethysmography, PPG) is a technology in which an optical sensor is used to sense changes in concentration of hemoglobin flowing in superficial blood vessels of a human body, to obtain pulses of the human body. Currently, more devices are equipped with PPG assemblies to collect PPG signals, and then a physiological parameter related to an arterial activity of a user, for example, blood oxygen, heart rate, respiratory rate, blood pressure, blood glucose, muscle oxygen, vascular resistance, or brain oxygen, is analyzed by using the PPG signals.

## SUMMARY

This application provides a physiological parameter detection method and a related apparatus, to improve accuracy of physiological parameter measurement.

According to a first aspect, an embodiment of this application provides a physiological parameter detection method. The method is applied to an electronic device including a photoplethysmography PPG assembly. The PPG assembly includes n (n≥2) detection modules, and each detection module includes one light source and one photoelectric detector. The method includes: collecting n PPG signals via the n detection modules, where each PPG signal is obtained in a manner in which a light source in one detection module emits a light ray toward skin and a photoelectric detector in the one detection module detects a light ray reflected from the skin, the n PPG signals include at least signals respectively collected by two detection modules with different spacings, and a spacing of a detection module is a distance between a light source and a photoelectric detector in the detection module; and determining a physiological parameter value of a user based on the n PPG signals, where the physiological parameter value is related to an arterial activity of the user.

The signals collected by the two detection modules with different spacings may include two PPG signals. One PPG signal is a signal collected by a detection module with a long spacing, and in the PPG signal, a proportion of a signal corresponding to a deep tissue is larger. The other PPG signal is a signal collected by a detection module with a short spacing, and in the PPG signal, a proportion of a signal corresponding to a superficial tissue is larger.

In this way, provided that the n collected PPG signals include signals collected by detection modules with different spacings, the electronic device may eliminate or suppress, by using a PPG signal collected by a detection module with a short spacing, a signal that corresponds to a detected tissue and that is in a PPG signal collected by a detection module with a long spacing, to improve accuracy of physiological parameter measurement.

For example, the PPG assembly may include a first detection module and a second detection module, and a spacing of the first detection module may be greater than a spacing of the second detection module. The electronic device may collect one PPG signal via the first detection module, and collect one PPG signal via the second detection module; eliminate or suppress, by using the PPG signal collected by the second detection module, a signal that corresponds to the superficial tissue and that is in the PPG signal collected by the first detection module; and then determine the physiological parameter value of the user by using a processed PPG signal collected by the first detection module.

With reference to the first aspect, in an implementation, before collecting the n PPG signals via the n detection modules, the method further includes: collecting a first PPG signal via one detection module in the n detection modules; and collecting the n PPG signals via the n detection modules specifically includes: when a physiological parameter value reflected by the first PPG signal is abnormal or a pulse reflected by the first PPG signal is incomplete, collecting the n PPG signals via the n detection modules.

In other words, when measuring a physiological parameter, the electronic device may first start only one detection module to measure the physiological parameter. When a detection effect is poor, the electronic device starts another detection module, and measures the physiological parameter value by using PPG signals collected by a plurality of detection modules. In this way, power consumption of the device can be reduced as much as possible when the accuracy of physiological parameter measurement is ensured.

With reference to the first aspect, in an implementation, determining the physiological parameter value based on the n PPG signals specifically includes: determining the physiological parameter value based on the n PPG signals and the first PPG signal.

In other words, when determining the physiological parameter value by using the plurality of PPG signals, the electronic device may also perform calculation with reference to the PPG signal collected previously when the physiological parameter is measured via only one detection module. In this way, an electronic device 100 can determine the physiological parameter of the user by using data collected by the electronic device as much as possible, thereby further improving the accuracy of physiological parameter measurement.

With reference to the first aspect, in an implementation, the physiological parameter value is a blood oxygen value, and in any detection module in the PPG assembly, light emitted by a light source includes light with at least two wavelengths; and determining the physiological parameter value of the user based on the n PPG signals specifically includes: calculating one ratio value R for each PPG signal in the n PPG signals, where the ratio value R is for reflecting a ratio between absorption rates of light with different wavelengths in the PPG signal; and determining the blood oxygen value of the user based on n ratio values R corresponding to the n PPG signals.

In other words, the solution may be applied to blood oxygen detection. Blood oxygen of the user is measured via a plurality of detection modules, to improve accuracy of blood oxygen measurement.

With reference to the first aspect, in an implementation, the blood oxygen value of the user is determined based on the n ratio values R corresponding to the n PPG signals: inputting the n ratio values R corresponding to the n PPG signals into a first blood oxygen model, to obtain the blood oxygen value of the user, where the first blood oxygen model is for representing a mathematical relationship between a plurality of ratio values and blood oxygen, the first blood oxygen model is determined based on a plurality of data sets, and one data set includes a blood oxygen value of a testee and k ratio values R calculated by using PPG signals collected by k (k≥1) detection modules from the measured person.

According to a second aspect, an embodiment of this application provides a physiological parameter detection method. The method is applied to an electronic device including a PPG assembly. The PPG assembly includes m (m≥2) groups of detection modules, one group of detection modules includes one or more detection modules, and one detection module includes one light source and one photoelectric detector. The method includes: collecting m groups of PPG signals via the m groups of detection modules, where each group of PPG signals includes one PPG signal collected by each detection module in one group of detection modules, one PPG signal is obtained in a manner in which a light source in one detection module emits a light ray toward skin and a photoelectric detector in the one detection module detects a light ray reflected from the skin, the m groups of PPG signals include a first group of PPG signals, the first group of PPG signals includes at least signals respectively collected by two detection modules with different spacings, and a spacing of a detection module is a distance between a light source and a photoelectric detector in the detection module; determining m physiological parameter values based on the m groups of PPG signals, where each physiological parameter value is determined based on one group of PPG signals; and determining a physiological parameter value of a user based on the m physiological parameter values, where the physiological parameter value is related to an arterial activity of the user.

The signals collected by the two detection modules with different spacings may include two PPG signals. One PPG signal is a signal collected by a detection module with a long spacing, and in the PPG signal, a proportion of a signal corresponding to a deep tissue is larger. The other PPG signal is a signal collected by a detection module with a short spacing, and in the PPG signal, a proportion of a signal corresponding to a superficial tissue is larger.

In this way, provided that a plurality of PPG signals collected by a group of detection modules include signals collected by detection modules with different spacings, the electronic device may eliminate or suppress, by using a PPG signal collected by a detection module with a short spacing, a signal that corresponds to a detected tissue and that is in a PPG signal collected by a detection module with a long spacing, to improve accuracy of physiological parameter calculation by using the PPG signals collected by the group of detection modules.

For example, the PPG assembly may include a first group of detection modules and a second group of detection modules. The first group of detection modules may include a first detection module and a second detection module, and a spacing of the first detection module may be greater than a spacing of the second detection module. The second group of detection modules may include one or more detection modules. The electronic device may collect one PPG signal via the first detection module, and collect one PPG signal via the second detection module; eliminate or suppress, by using the PPG signal collected by the second detection module, a signal that corresponds to the superficial tissue and that is in the PPG signal collected by the first detection module; and then determine one physiological parameter value corresponding to the first group of detection modules by using a processed PPG signal collected by the first detection module. Similarly, the electronic device determines one physiological parameter value corresponding to the second group of detection modules by using one or more PPG signals collected by the second group of detection modules. Then, the electronic device may determine the physiological parameter value of the user based on the physiological parameter value corresponding to the first group of detection modules and the physiological parameter value corresponding to the second group of detection modules.

It can be learned that the physiological parameter value of the user is finally determined by using a plurality of physiological parameter values collected by a plurality of groups of detection modules. This can mitigate impact of a PPG wearing position on accuracy of physiological parameter measurement as much as possible, to improve accuracy and stability of physiological parameter measurement.

With reference to the second aspect, in an implementation, two groups of detection modules in the PPG assembly include at least a same light source and/or at least a same photoelectric detector.

In other words, a device between different groups of detection modules may be shared, to reduce device costs and power consumption.

With reference to the second aspect, in an implementation, the physiological parameter value of the user is a largest value, a smallest value, an average value, a median, or a mode of the m physiological parameter values.

In other words, a final physiological parameter status of the user may be comprehensively considered by using the plurality of physiological parameter values, to reduce an error in evaluation on the physiological parameter of the user.

With reference to the second aspect, in an implementation, the physiological parameter value is a blood oxygen value, and in any detection module in the PPG assembly, light emitted by a light source includes light of at least two wavelengths; and one blood oxygen value in the m blood oxygen values is determined based on one or more ratio values R corresponding to one group of PPG signals, one ratio value R is determined based on one PPG signal in one group of PPG signals, and the ratio value R is for reflecting a ratio between absorption rates of light with different wavelengths in the PPG signal.

In other words, the solution may be applied to blood oxygen detection. Blood oxygen of the user is measured via a plurality of detection modules, to improve accuracy of blood oxygen measurement.

With reference to the second aspect, in an implementation, the one blood oxygen value is obtained by inputting the one or more ratio values R into a first blood oxygen model, the first blood oxygen model is for representing a mathematical relationship between a plurality of ratio values and blood oxygen, the first blood oxygen model is determined based on a plurality of data sets, and one data set includes a blood oxygen value of a testee and k ratio values R calculated by using PPG signals collected by k ($k \geq 1$) detection modules from the measured person.

With reference to the second aspect, in an implementation, before collecting the m groups of PPG signals via the m groups of detection modules, the method further includes: collecting a first PPG signal via one detection module in the m groups of detection modules; and collecting the m groups of PPG signals via the m groups of detection modules specifically includes: when a physiological parameter value reflected by the first PPG signal is abnormal or a pulse reflected by the first PPG signal is incomplete, collecting the m groups of PPG signals via the m groups of detection modules.

In other words, when measuring a physiological parameter, the electronic device may first start only one detection module to measure the physiological parameter. When a detection effect is poor, the electronic device starts another detection module, and measures the physiological parameter value by using PPG signals collected by a plurality of groups of detection modules. In this way, power consumption of the device can be reduced as much as possible when the accuracy of physiological parameter measurement is ensured.

With reference to the second aspect, in an implementation, the determining the physiological parameter value of the user based on the m physiological parameter values corresponding to the m groups of PPG signals specifically includes: determining the physiological parameter value of the user based on the m physiological parameter values corresponding to the m groups of PPG signals and the first PPG signal.

In other words, when determining the physiological parameter value by using the plurality of PPG signals, the electronic device may also perform calculation with reference to the PPG signal collected previously when the physiological parameter is measured via only one detection module. In this way, an electronic device 100 can determine the physiological parameter of the user by using data collected by the electronic device as much as possible, thereby further improving the accuracy of physiological parameter measurement.

With reference to the first aspect and the second aspect, in an implementation, the method further includes: when the physiological parameter value reflected by the first PPG signal is normal or the pulse reflected by the first PPG signal is complete, determining that the physiological parameter value determined based on the first PPG signal is the physiological parameter value of the user.

In other words, if the electronic device can accurately measure the physiological parameter of the user via only one detection module, there is no need to start a plurality of detection modules, to reduce power consumption and a computation amount of the electronic device.

With reference to the first aspect and the second aspect, in an implementation, the physiological parameter value is a value of a first physiological parameter, and the first physiological parameter includes any one or more of the following: blood oxygen, heart rate, respiratory rate, blood pressure, blood glucose, muscle oxygen, vascular resistance, or brain oxygen.

With reference to the first aspect and the second aspect, in an implementation, two detection modules in the PPG assembly include at least a same light source or at least a same photoelectric detector.

In other words, a device between different detection modules may be shared, to reduce device costs and power consumption.

According to a third aspect, an embodiment of this application provides an electronic device, including a PPG assembly, a memory, one or more processors, and one or more programs. The PPG assembly includes n (n≥2) detection modules, each detection module includes one light source and one photoelectric detector, and when the one or more processors execute the one or more programs, the electronic device is caused to implement the method according to any one of the first aspect or the implementations of the first aspect.

According to a fourth aspect, an embodiment of this application provides an electronic device, including a PPG assembly, a memory, one or more processors, and one or more programs. The PPG assembly includes m (m≥2) groups of detection modules, each group of detection modules includes one or more detection modules, each detection module includes one light source and one photoelectric detector, and when the one or more processors execute the one or more programs, the electronic device is caused to implement the method according to any one of the second aspect or the implementations of the second aspect.

According to a fifth aspect, an embodiment of this application provides a computer-readable storage medium, including instructions. When the instructions are run on an electronic device, the electronic device is caused to perform the method according to any one of the first aspect or the implementations of the first aspect, or the method according to any one of the second aspect or the implementations of the second aspect.

According to a sixth aspect, an embodiment of this application provides a computer program product. When the computer program product is run on a computer, the computer is caused to perform the method according to any one of the first aspect or the implementations of the first aspect, or the method according to any one of the second aspect or the implementations of the second aspect.

## BRIEF DESCRIPTION OF DRAWINGS

FIG. 1A is a diagram of a principle of blood oxygen detection;
FIG. 1B is a diagram of a composition principle of a collected PPG signal during blood oxygen detection;
FIG. 2 is a diagram of a principle of blood oxygen detection according to an embodiment of this application;
FIG. 3A is a diagram of a hardware structure of an electronic device 100 according to an embodiment of this application;
FIG. 3B is a diagram of a structure of a PPG assembly 193 according to an embodiment of this application;
FIG. 4 is a schematic flowchart of a physiological parameter detection method according to an embodiment of this application;
FIG. 5 is a diagram of a PPG signal according to an embodiment of this application;
FIG. 6 is a detailed flowchart of a method for determining a blood oxygen value by using n PPG signals according to an embodiment of this application;
FIG. 7 is a diagram of a structure of a PPG assembly according to an embodiment of this application;
FIG. 8 is a diagram of another structure of a PPG assembly according to an embodiment of this application;
FIG. 9 is a diagram of another structure of a PPG assembly according to an embodiment of this application;
FIG. 10 is a diagram of another structure of a PPG assembly according to an embodiment of this application;
FIG. 11 is a diagram of another structure of a PPG assembly according to an embodiment of this application;
FIG. 12 is a diagram of another structure of a PPG assembly according to an embodiment of this application;
FIG. 13 is a schematic flowchart of another physiological parameter detection method according to an embodiment of this application;
FIG. 14 is a diagram of another structure of a PPG assembly according to an embodiment of this application;
FIG. 15 is a diagram of another structure of a PPG assembly according to an embodiment of this application;
FIG. 16 is a diagram of another structure of a PPG assembly according to an embodiment of this application;
FIG. 17 is a diagram of another structure of a PPG assembly according to an embodiment of this application;
FIG. 18 is a diagram of another structure of a PPG assembly according to an embodiment of this application;
FIG. 19 is a diagram of another structure of a PPG assembly according to an embodiment of this application;
FIG. 20 is a diagram of another structure of a PPG assembly according to an embodiment of this application;
FIG. 21 is a schematic flowchart of another physiological parameter detection method according to an embodiment of this application;
FIG. 22 is a schematic flowchart of another physiological parameter detection method according to an embodiment of this application; and
FIG. 23 is a diagram of a structure of a physiological parameter detection apparatus 200 according to an embodiment of this application.

**DESCRIPTION OF EMBODIMENTS**

Technical solutions in embodiments of this application are clearly and completely described in the following with reference to accompanying drawings. In the descriptions of embodiments of this application, "/" indicates or, unless otherwise specified. For example, A/B may indicate A or B. In this specification, "and/or" describes only an association relationship between associated objects, and indicates that three relationships may exist. For example, A and/or B may indicate the following three cases: Only A exists, both A and B exist, and only B exists. In addition, in the descriptions of embodiments of this application, "a plurality of" means two or more than two.

The following terms "first" and "second" are merely intended for a purpose of description, and shall not be understood as an indication or implication of relative importance or implicit indication of a quantity of indicated technical features. Therefore, a feature limited by "first" and "second" may explicitly or implicitly include one or more features. In the descriptions of embodiments of this application, unless otherwise specified, "a plurality of" means two or more.

A term "user interface (user interface, UI)" in the following embodiments of this application is a medium interface for interaction and information exchange between an application or an operating system and a user, and implements conversion between an internal form of information and a form acceptable to the user. The user interface is source code written in a specific computer language like Java or an extensible markup language (extensible markup language, XML). Interface source code is parsed and rendered on an electronic device, and is finally presented as content that can be identified by the user. The user interface is usually represented in a form of a graphical user interface (graphical user interface, GUI), and is a user interface that is related to a computer operation and that is displayed in a graphic manner. The user interface may be a visual interface element like a text, an icon, a button, a menu, a tab, a text box, a dialog box, a status bar, a navigation bar, or a Widget that is displayed on a display of the electronic device.

Blood oxygen, also referred to as blood oxygen saturation ($SpO_2$), is oxygen in blood, measures a capability of transporting oxygen by blood, and is one of important indicators of a function of a respiratory circulation system. Accurate monitoring of a blood oxygen value is of important value for human health evaluation.

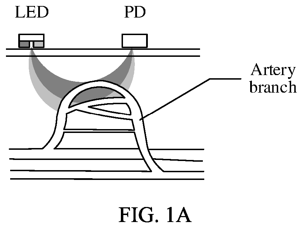

**FIG. 1A is a diagram of a principle of blood oxygen detection.**

As shown in FIG. 1A, currently, blood oxygen detection is mainly implemented via a PPG assembly. The PPG assembly includes a light source like a light-emitting diode (light-emitting diode, LED) shown in FIG. 1A and a photoelectric detector (photoelectric detector, PD). A spacing with a specific distance exists between the light source and the photoelectric detector. The light source emits a light ray toward skin. The light passes through a skin tissue and is absorbed and scattered by a tissue, and finally returns to a skin surface layer and is detected by the photoelectric detector to obtain a PPG signal, so that a blood oxygen value is calculated based on the PPG signal.

It can be learned that one light source and one photoelectric detector work together in pair, one light source emits a light ray, and one photoelectric detector is configured to detect a light ray finally reflected after the light ray emitted by the light source is absorbed by the skin, to obtain a PPG signal. For ease of description, the light source and the photoelectric detector in pair are collectively referred to as one detection module in the following. It should be understood that the name does not constitute a limitation on embodiments of this application.

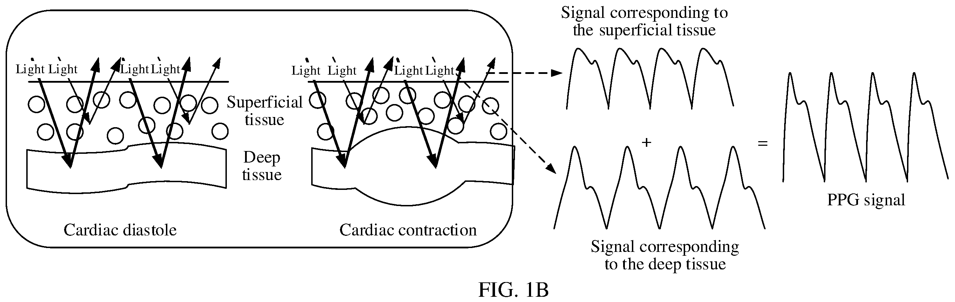

**For example, FIG. 1B is a diagram of a composition principle of a collected PPG signal during blood oxygen detection.**

As shown in FIG. 1B, after passing through a skin tissue, a light ray emitted by a light source is absorbed and scattered by a superficial tissue and a deep tissue. Finally, after passing through the superficial tissue and returning to a skin surface layer, the light ray is detected by a photoelectric detector, to collect a signal corresponding to the superficial tissue. After passing through the deep tissue and returning to the skin surface layer, the light ray is detected by the photoelectric detector, to collect a signal corresponding to the deep tissue. The signal corresponding to the superficial tissue and the signal corresponding to the deep tissue finally form a PPG signal collected by the photoelectric detector.

Because the superficial tissue usually does not have an artery, the signal corresponding to the superficial tissue is a signal corresponding to a tissue like a vein or a capillary, does not include or includes less pulse blood oxygen information, and cannot be used to analyze a blood oxygen status of a user. Because the deep tissue includes a dermal arteriole, the signal corresponding to the deep tissue is a target signal for blood oxygen detection, and can be used to analyze the blood oxygen status of the user.

However, a light ray emitted by an existing PPG assembly inevitably passes through a superficial tissue of skin. Therefore, an obtained PPG signal definitely includes an interference signal from the superficial tissue. In this case, if the PPG signal collected by a photoelectric detector is directly used to analyze blood oxygen of the user, accuracy of detection of blood oxygen of the user is affected.

In addition, in daily use, factors such as inappropriate wearing, movement of a wearer, and a low temperature may cause an increase in a proportion of a signal corresponding to the superficial tissue in the PPG signal. When the proportion of the signal corresponding to the superficial tissue is excessively large, the PPG signal is abnormal, further reducing accuracy of blood oxygen detection.

In a detection module, a longer spacing between a light source and a photoelectric detector indicates a deeper tissue depth that can be reached by light. On the contrary, a shorter spacing between the light source and the photoelectric detector indicates a shallower tissue depth that can be reached by the light.

Therefore, in an embodiment of this application, a PPG assembly including at least two detection modules is designed. In the two detection modules, spacings between light sources and photoelectric detectors are different, so that PPG signals obtained by the two detection modules include signals that correspond to a superficial tissue and a deep tissue and that are in different proportions.

In a PPG signal obtained by a detection module with a long spacing, a proportion of a signal corresponding to the deep tissue is larger. In a PPG signal obtained by a detection module with a short spacing, a proportion of a signal corresponding to the superficial tissue is larger. Therefore, interference from an interference signal in the PPG signal collected by the detection module with the long spacing may be excluded or reduced by using the PPG signal obtained by the detection module with the short spacing, to implement more accurate blood oxygen detection.

For example, a spacing greater than a threshold is a long spacing, and a spacing less than the threshold is a short spacing. In this embodiment of this application, the threshold may be 4 mm. In other words, the long spacing may be a spacing greater than 4 mm, and the short spacing may be a spacing less than 4 mm. The threshold is not limited in this embodiment of this application.

It may be understood that, in addition to that a blood oxygen value may be calculated by using a PPG signal obtained by a reflective PPG assembly during blood oxygen detection, some other physiological parameters such as heart rate, respiratory rate, blood pressure, blood glucose, muscle oxygen, vascular resistance, and brain oxygen may also be measured by using a PPG signal reflected from a skin tissue. Therefore, all physiological parameters related to an arterial activity may be detected via the PPG assembly designed in this embodiment of this application.

It can be learned that an embodiment of this application provides a physiological parameter detection method. In the method, a plurality of PPG signals can be collected via the foregoing PPG assembly including a plurality of detection modules, and then a physiological parameter value of a user is calculated by using the plurality of PPG signals, to improve accuracy of physiological parameter measurement.

It should be noted that different detection modules may share a same light source or a same photoelectric detector. Therefore, the PPG assembly provided in this embodiment of this application may include one or more light sources and a plurality of photoelectric detectors, or a plurality of light sources and one or more photoelectric detectors.

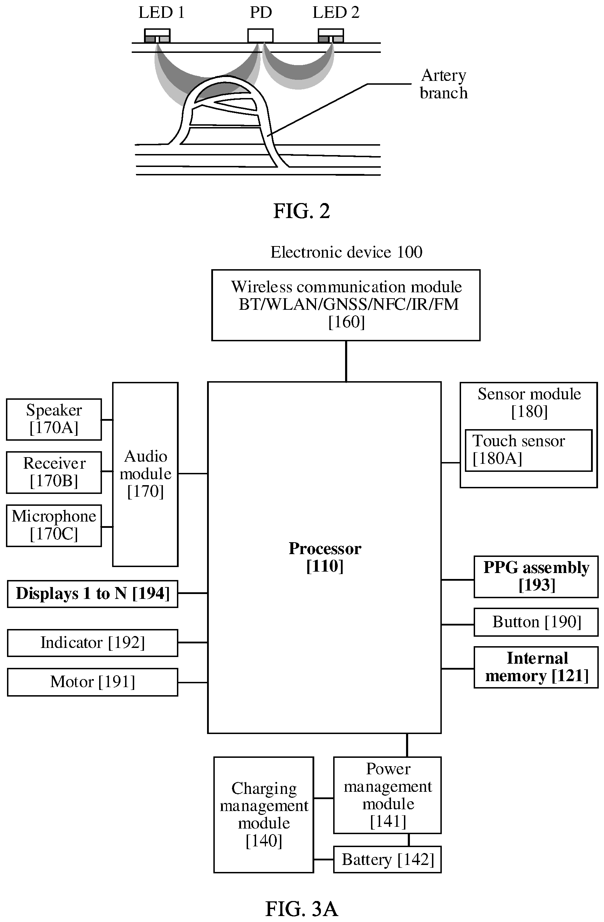

For example, FIG. 2 is a diagram of a principle of blood oxygen detection according to an embodiment of this application.

As shown in FIG. 2, the PPG assembly may include two light sources (an LED 1 and an LED 2) and one photoelectric detector (PD). A spacing between the LED 1 and the PD is greater than a spacing between the LED 2 and the PD. The LED 1 and the PD form one detection module, and the LED 2 and the PD form one detection module.

In this case, in an actual process of measuring blood oxygen, the LED 1 and the LED 2 may be respectively turned on at different time points, so that the PD can collect, at different time points, light rays reflected for different light sources, to obtain PPG signals. An obtained PPG signal (hereinafter referred to as a PPG signal 1) corresponding to a light ray reflected for the LED 1 includes a signal that corresponds to the deep tissue and that has a larger proportion. An obtained PPG signal (hereinafter referred to as a PPG signal 2) corresponding to a light ray reflected for the LED 2 includes a signal that corresponds to the superficial tissue and that has a larger proportion. Then, the blood oxygen value of the user may be calculated by using the PPG signal 1 and the PPG signal 2.

It may be understood that the PPG assembly shown in FIG. 2 is merely an example. In another embodiment of this application, there may be another case for quantities and positions of light sources and photoelectric detectors. In addition, in FIG. 2, blood oxygen is used as an example to describe a principle of blood oxygen detection. For other physiological parameters, such as heart rate, respiratory rate, blood pressure, blood glucose, muscle oxygen, vascular resistance, and brain oxygen, there are similar measurement principles. Details are not described herein again.

FIG. 3A is a diagram of a hardware structure of an electronic device 100 according to an embodiment of this application.

The electronic device 100 may be a mobile phone, a tablet computer, a desktop computer, a laptop computer, a handheld computer, a notebook computer, an ultra-mobile personal computer (ultra-mobile personal computer, UMPC), a netbook, a cellular phone, a personal digital assistant (personal digital assistant, PDA), an augmented reality (augmented reality, AR) device, a virtual reality (virtual reality, VR) device, an artificial intelligence (artificial intelligence, AI) device, a wearable device, a vehicle-mounted device, a smart home device, and/or a smart city device. A specific type of the electronic device is not limited in embodiments of this application.

Preferably, in this embodiment of this application, the electronic device 100 may be a wearable device worn by a user, for example, a watch, a band, or a ring.

The electronic device 100 may include a processor 110, an internal memory 121, a charging management module 140, a power management module 141, a battery 142, a sensor module 180, a PPG assembly 193, a display 194, and the like. Optionally, the electronic device 100 may further include any one or more of the following: a wireless communication module 160, an audio module 170, a button 190, a motor 191, an indicator 192, and the like. The audio module 170 may include any one or more of the following: a speaker 170A, a receiver 170B, and a microphone 170C. The sensor module 180 may include a touch sensor 180A and the like.

The processor 110 may include one or more processing units. For example, the processor 110 may include an application processor (application processor, AP), a modem processor, a graphics processing unit (graphics processing unit, GPU), an image signal processor (image signal processor, ISP), a controller, a video codec, a digital signal processor (digital signal processor, DSP), a baseband processor, a neural network processing unit (neural network processing unit, NPU), and/or the like. Different processing units may be independent devices, or may be integrated into one or more processors.

In some implementations, the processor 110 may be configured to control the PPG assembly 193 to collect a PPG signal, and calculate a physiological parameter value of the user based on the collected PPG signal.

The controller may generate an operation control signal based on an instruction operation code and a time sequence signal, to complete control of instruction reading and instruction execution.

A memory may be further disposed in the processor 110, and is configured to store instructions and data. In some embodiments, the memory in the processor 110 is a cache. The memory may store instructions or data just used or cyclically used by the processor 110. If the processor 110 needs to use the instructions or the data again, the processor may directly invoke the instructions or the data from the memory. This avoids repeated access, reduces waiting time of the processor 110, and improves system efficiency.

In some embodiments, the processor 110 may include one or more interfaces. The interface may include an inter-integrated circuit (inter-integrated circuit, I2C) interface, an inter-integrated circuit sound (inter-integrated circuit sound, I2S) interface, a pulse code modulation (pulse code modulation, PCM) interface, a universal asynchronous receiver/transmitter (universal asynchronous receiver/transmitter, UART) interface, a mobile industry processor interface (mobile industry processor interface, MIPI), a general-purpose input/output (general-purpose input/output, GPIO) interface, a subscriber identity module (subscriber identity module, SIM) interface, a universal serial bus (universal serial bus, USB) interface, and/or the like.

The charging management module 140 is configured to receive a charging input from a charger. The charger may be a wireless charger or a wired charger. In some wired charging embodiments, the charging management module 140 may receive a charging input from the wired charger. In some wireless charging embodiments, the charging management module 140 may receive a wireless charging input through a wireless charging coil of the electronic device 100. The charging management module 140 supplies power to the electronic device via the power management module 141 when charging the battery 142.

The power management module 141 is configured to be connected to the battery 142, the charging management module 140, and the processor 110. The power management module 141 receives an input from the battery 142 and/or the charging management module 140, and supplies power to the processor 110, the internal memory 121, the display 194, the wireless communication module 160, and the like. The power management module 141 may be further configured to monitor parameters such as a battery capacity, a battery cycle count, and a battery health status (electric leakage or impedance). In some other embodiments, the power management module 141 may alternatively be disposed in the processor 110. In some other embodiments, the power management module 141 and the charging management module 140 may alternatively be disposed in a same device.

The wireless communication module 160 may provide a wireless communication solution that is applied to the electronic device 100 and that includes a wireless local area network (wireless local area network, WLAN) (for example, a wireless fidelity (wireless fidelity, Wi-Fi) network), Bluetooth (Bluetooth, BT), a global navigation satellite system (global navigation satellite system, GNSS), frequency modulation (frequency modulation, FM), a near field communication (near field communication, NFC) technology, an infrared (infrared, IR) technology, a nearlink (NearLink), intra-body communication (intra-body communication, IBC), or the like. For example, when two electronic devices communicate by using an intra-body communication solution, the two electronic devices each have at least one electrode in contact with skin. By using the foregoing electrode in contact with the skin, the two electronic devices receive and send information to each other through a human body. The wireless communication module 160 may be one or more devices integrating at least one communication processor module.

The electronic device 100 may implement a display function through the GPU, the display 194, the application processor, and the like. The GPU is a microprocessor for image processing, and is connected to the display 194 and the application processor. The GPU is configured to perform mathematical and geometric computation, and render an image. The processor 110 may include one or more GPUs, which execute program instructions to generate or change display information.

The display 194 is configured to display an image, a video, and the like. The display 194 includes a display panel. The display panel may be a liquid crystal display (liquid crystal display, LCD), an organic light-emitting diode (organic light-emitting diode, OLED), an active-matrix organic light-emitting diode (active-matrix organic light-emitting diode, AMOLED), a flexible light-emitting diode (flex light-emitting diode, FLED), a MiniLed, a MicroLed, a Micro-oLed, a quantum dot light emitting diode (quantum dot light emitting diode, QLED), or the like. In some embodiments, the electronic device 100 may include one or N displays 194, where N is a positive integer greater than 1.

In some implementations, the display 194 may be configured to display the physiological parameter value of the user.

The internal memory 121 may include one or more random access memories (random access memory, RAM) and one or more nonvolatile memories (nonvolatile memory, NVM). The random access memory may be directly read and written by the processor 110, may be configured to store executable programs (such as machine instructions) of an operating system or another running program, and may also be configured to store data of a user and an application. The nonvolatile memory may also store the executable programs, the data of the user and the application, and the like, and may be loaded into the random access memory in advance, to be directly read and written by the processor 110.

In some implementations, the internal memory 121 may be configured to store the PPG signal collected by the PPG assembly 193 and the physiological parameter value that is of the user and that is calculated by the processor 110.

The electronic device 100 may implement audio functions, such as music playing and recording, via the audio module 170, the speaker 170A, the receiver 170B, the microphone 170C, the application processor, and the like.

The audio module 170 is configured to convert digital audio information into an analog audio signal for output, and is also configured to convert an analog audio input into a digital audio signal. The audio module 170 may be further configured to code and decode an audio signal. In some embodiments, the audio module 170 may be disposed in the processor 110, or some functional modules in the audio module 170 are disposed in the processor 110.

The speaker 170A, also referred to as a "loudspeaker", is configured to convert an audio electrical signal into a sound signal. The electronic device 100 may be used to listen to music or answer a call in a hands-free mode over the speaker 170A.

The receiver 170B, also referred to as an "earpiece", is configured to convert an electrical audio signal into a sound signal. When a call is answered or speech information is received through the electronic device 100, the receiver 170B may be put close to a human ear to listen to a speech.

The microphone 170C, also referred to as a "mike" or a "mic", is configured to convert a sound signal into an electrical signal. When making a call or sending speech information, a user may make a sound near the microphone 170C through the mouth of the user, to input a sound signal to the microphone 170C. At least one microphone 170C may be disposed in the electronic device 100. In some other embodiments, two microphones 170C may be disposed in the electronic device 100, to collect a sound signal and implement a noise reduction function. In some other embodiments, three, four, or more microphones 170C may alternatively be disposed in the electronic device 100, to collect a sound signal, implement noise reduction, and identify a sound source, to implement a directional recording function and the like.

The touch sensor 180A is also referred to as a "touch device". The touch sensor 180A may be disposed on the display 194, and the touch sensor 180A and the display 194 form a touchscreen, which is also referred to as a "touch screen". The touch sensor 180A is configured to detect a touch operation performed on or near the touch sensor 180A. The touch sensor may transfer the detected touch operation to the application processor to determine a type of a touch event. A visual output related to the touch operation may be provided through the display 194. In some other embodiments, the touch sensor 180A may alternatively be disposed on a surface of the electronic device 100 at a position different from that of the display 194.

The button 190 includes a power button, a volume button, and the like. The button 190 may be a mechanical button, or may be a touch button. The electronic device 100 may receive a key input, and generate a key signal input related to a user setting and function control of the electronic device 100.

The motor 191 may generate a vibration prompt. The motor 191 may be configured to provide an incoming call vibration prompt and a touch vibration feedback. For example, touch operations performed on different applications (for example, photographing and audio playback) may correspond to different vibration feedback effects. The motor 191 may also correspond to different vibration feedback effects for touch operations performed on different areas of the display 194. Different application scenarios (for example, a time reminder, information receiving, an alarm clock, and a game) may also correspond to different vibration feedback effects. A touch vibration feedback effect may be further customized.

The indicator 192 may be an indicator light, and may be configured to indicate a charging status and a power change, or may be configured to indicate a message, a missed call, a notification, and the like.

The PPG assembly 193 may be configured to collect the PPG signal. The PPG signal may be for calculating a value of a physiological parameter related to a cardiovascular system function status of the user. For example, the physiological parameter may be blood oxygen, heart rate, respiratory rate, blood pressure, or the like.

The PPG assembly 193 may include one or more light sources and a plurality of photoelectric detectors, or a plurality of light sources and one or more photoelectric detectors. One light source and one photoelectric detector may form one detection module.

In this embodiment of this application, the PPG assembly 193 may include n (n≥2) detection modules, and a photoelectric detector in each detection module may be configured to collect a PPG signal corresponding to a light source in the detection module.

In addition, the n detection modules include at least detection modules with two spacings, and there may be one or more detection modules with each spacing. A spacing of a detection module is a distance between a light source and a photoelectric detector in the detection module.

The light source may be a light-emitting diode or a laser. The light source may emit a light ray. After the light ray is irradiated on skin, the photoelectric detector detects remaining reflected light after the light ray is absorbed by blood and a tissue in a penetration process, and converts the remaining reflected light into an electrical signal, to obtain a PPG signal. Intensity of the reflected light changes with arterial pulsation, and the PPG signal also follows the arterial pulsation, that is, heartbeats of the user rhythmically fluctuate. Therefore, a physiological parameter of the user, for example, blood oxygen, heart rate, respiratory rate, blood pressure, blood glucose, muscle oxygen, vascular resistance, or brain oxygen, may be calculated by using the PPG signal.

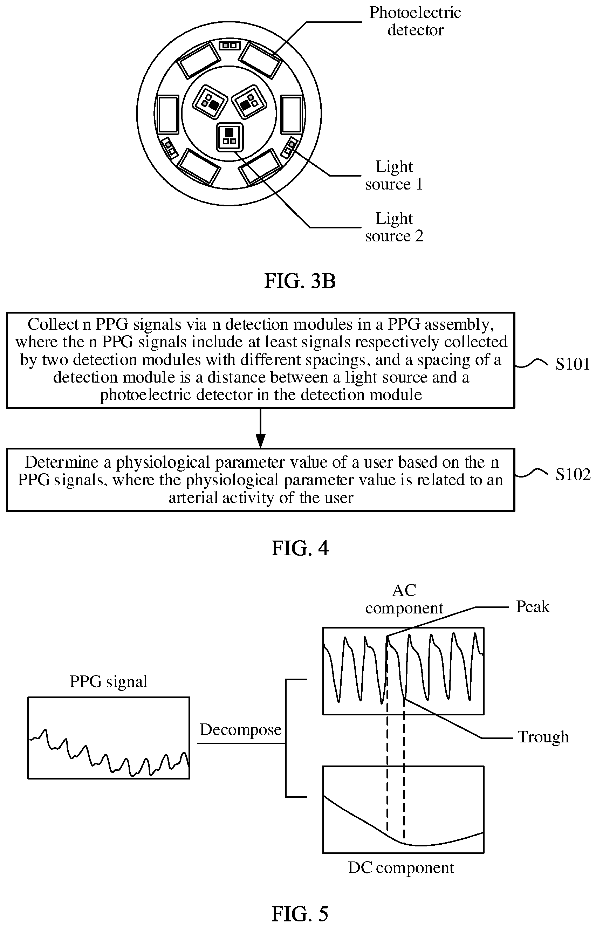

For example, FIG. 3B is a diagram of a structure of a PPG assembly 193 according to an embodiment of this application.

As shown in FIG. 3B, the PPG assembly 193 includes six light sources and six photoelectric detectors in total. Three light sources are located in an inner ring, and form three vertices of a triangle in position. The other three light sources and the six photoelectric detectors are located in an outer ring, and are placed around a center point of the PPG assembly.

Further, a light source located in the inner ring (for example, a light source 2 in FIG. 3B) may be different from a light source located in the outer ring (for example, a light source 1 in FIG. 3B). A light ray that can be emitted by the light source 1 may include red light and infrared light, and a light ray that can be emitted by the light source 2 may include red light, infrared light, and green light.

In this embodiment of this application, if a measured physiological parameter is blood oxygen, a light source that emits red light and infrared light may be used. If the measured physiological parameter is heart rate, a light source that emits green light may be used. It can be learned that when different physiological parameters are measured, the PPG assembly may use light sources with different wavelengths.

It may be understood that FIG. 3B is merely a diagram of a possible PPG assembly according to an embodiment of this application. In another embodiment of this application, the PPG assembly may alternatively present another structure, and a light source in the PPG assembly may alternatively emit light rays with more or fewer wavelengths. This is not limited in this embodiment of this application.

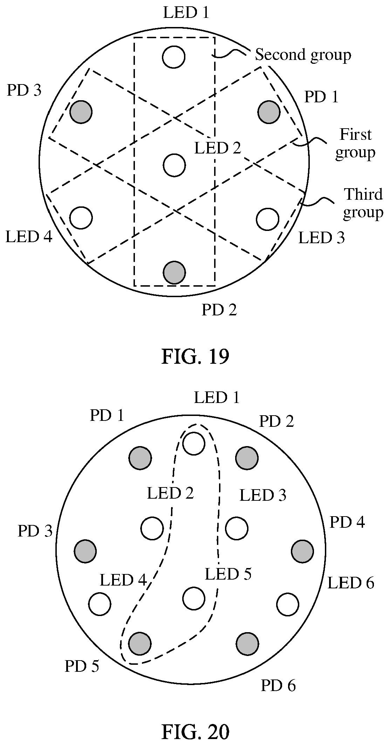

For a specific working principle of the PPG assembly 193 shown in FIG. 3B, refer to subsequent content in FIG. 20. Details are not described herein.

In some embodiments, the sensor module 180 in the electronic device 100 may further include any one or more of the following sensors: a pressure sensor, an acceleration sensor, a barometric pressure sensor, a temperature sensor, a gyroscope sensor, and the like.

The pressure sensor is configured to sense a pressure signal, and convert the pressure signal into an electrical signal. In some implementations, the pressure sensor may be disposed on the display 194. The electronic device 100 may also calculate a touch position based on a detection signal of the pressure sensor. In some embodiments, touch operations that are performed in a same touch position but have different touch operation intensity may correspond to different operation instructions.

The acceleration sensor may detect magnitudes of accelerations of the electronic device 100 in various directions (generally on three axes). When the electronic device 100 is still, a magnitude and a direction of gravity may be detected. The acceleration sensor may be further configured to identify a posture of the electronic device, and is used in an application like switching between a landscape mode and a portrait mode or a pedometer.

The barometric pressure sensor may be configured to measure barometric pressure. In some embodiments, the barometric pressure sensor may also be configured to measure water pressure.

The temperature sensor may be configured to measure a body temperature of the user, or may be configured to measure a temperature of an environment in which the user is located.

The gyroscope sensor may be configured to determine a moving posture of the electronic device 100. In some embodiments, an angular velocity of the electronic device 100 around three axes (namely, axes x, y, and z) may be determined by using the gyroscope sensor.

It may be understood that the structure shown in embodiments of this application does not constitute a specific limitation on the electronic device 100. In some other embodiments of this application, the electronic device 100 may include more or fewer components than those shown in the figure, or some components may be combined, or some components may be split, or different component arrangements may be used. The components shown in the figure may be implemented by hardware, software, or a combination of software and hardware.

**FIG. 4 is a schematic flowchart of a physiological parameter detection method according to an embodiment of this application.**

**S101:** An electronic device 100 collects n PPG signals via n detection modules in a PPG assembly, where the n PPG signals include at least signals respectively collected by two detection modules with different spacings, and a spacing of a detection module is a distance between a light source and a photoelectric detector in the detection module.

The electronic device 100 may be a wearable device worn by a user, for example, a watch, a band, or a ring.

The electronic device 100 may include the PPG assembly, and the PPG assembly may be the PPG assembly 193 in FIG. 3A.

The PPG assembly may include n (n≥2) detection modules. Each detection module may include one light source and one photoelectric detector. The photoelectric detector in the detection module may be configured to collect one PPG signal corresponding to the light source in the detection module.

In some implementations, devices included in different detection modules may be independent of each other, or a device included in different detection modules may be shared. In this case, if the n detection modules include two detection modules that share a device, the two detection modules may include at least a same light source or at least a same photoelectric detector. The device included in the different detection modules is shared, to help reduce costs and power consumption.

That the electronic device 100 collects one PPG signal via one detection module may specifically include: The electronic device 100 controls a light source, for example, a light-emitting diode, in the detection module to emit a light ray toward skin, then detects a light ray reflected from the skin via a photoelectric detector in the detection module, and converts the light ray into an electrical signal, to obtain the PPG signal.

When a distance between a light source and a photoelectric detector in a detection module is different, a skin tissue depth that can be reached by a light ray emitted by the light source is different. Therefore, proportions of signals that are reflected from a superficial tissue and a deep tissue and that are included in a PPG signal detected by the photoelectric detector are different.

The shorter the distance between the light source and the photoelectric detector, the shallower the skin tissue depth that can be reached by the light ray emitted by the light source. In this way, a proportion of a signal that corresponds to the superficial tissue and that is included in the PPG signal detected by the photoelectric detector is greater than a proportion of a signal corresponding to the deep tissue. The longer the distance between the light source and the photoelectric detector, the deeper the skin tissue depth that can be reached by the light ray emitted by the light source. In this way, the proportion of the signal that corresponds to the deep tissue and that is included in the PPG signal detected by the photoelectric detector is greater than the proportion of the signal corresponding to the superficial tissue.

In this case, because the n detection modules include at least two detection modules with different spacings, the n PPG signals collected by the electronic device 100 include at least two PPG signals that have different compositions. The composition may be proportions of signals corresponding to tissues of different depths.

For example, step S101 may be performed in a plurality of cases in the following.

(1) After receiving an operation of detecting a physiological parameter by the user, the electronic device 100 may trigger step S101 to be performed.

The electronic device 100 may provide a user interface for measuring the physiological parameter, so that the user may enable or disable physiological parameter measurement based on the user interface, so that the user can learn of a body status of the user at any time.

(2) The electronic device 100 may trigger, when a preset condition is met, step S101 to be performed.

The preset condition may be: Preset time is reached, it is detected that the user is in a still state, or the like.

In this way, the electronic device 100 may automatically identify an optimal occasion for physiological parameter measurement, and automatically trigger physiological parameter measurement, to provide an intelligent and convenient physiological parameter monitoring service for the user.

It may be understood that the electronic device 100 may alternatively trigger, in another case, PPG signal collection. This is not limited in this embodiment of this application.

In some implementations, the light source may emit different light rays due to different detected physiological parameters. The electronic device 100 may select a light ray emitted by the light source based on a measured physiological parameter. For example, if the detected physiological parameter is blood oxygen, the light ray emitted by the light source may include red light and infrared light. If the detected physiological parameter is heart rate, the light ray emitted by the light source may be green light.

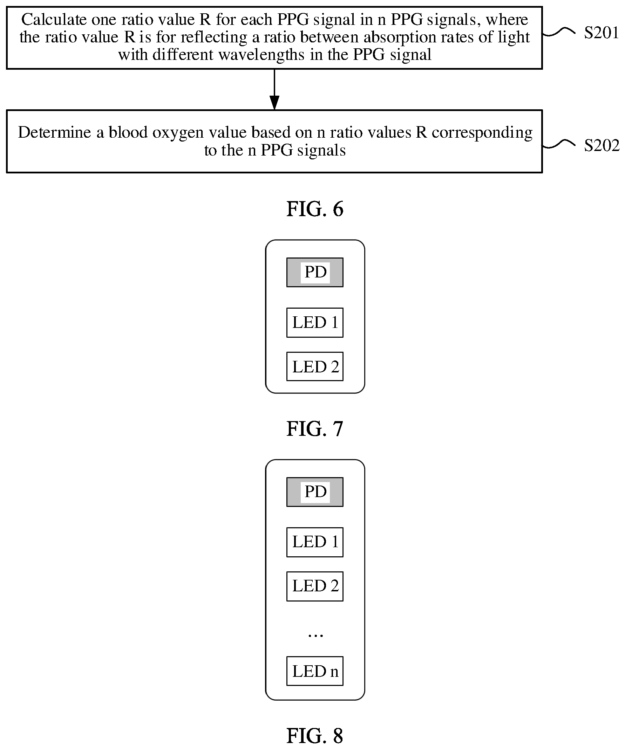

It should be noted that when the electronic device 100 collects PPG signals via the n detection modules, the electronic device 100 may simultaneously start the n detection modules to collect the n PPG signals, or may respectively start different detection modules at different time points to collect the PPG signals. This may be specifically determined by a layout of light sources and photoelectric detectors in the n detection modules. For details about how the electronic device 100 starts the n detection modules, refer to related content in PPG assemblies specifically enumerated in FIG. 7 to FIG. 12 subsequently. Details are not described herein.

**S102:** The electronic device 100 determines a physiological parameter value of the user based on the n PPG signals, where the physiological parameter value is related to an arterial activity of the user.

The physiological parameter value may be a value of a first physiological parameter of the user. For example, the first physiological parameter may be any one or more of the following: blood oxygen, heart rate, respiratory rate, blood pressure, blood glucose, muscle oxygen, vascular resistance, brain oxygen, or the like.

A proportion of a signal that corresponds to the deep tissue and that is included in a PPG signal collected by a detection module with a long spacing is larger, and a proportion of a signal that corresponds to the superficial tissue and that is included in a PPG signal collected by a detection module with a short spacing is larger. Therefore, when determining the physiological parameter value, the electronic device 100 may remove or suppress an interference signal in the PPG signal corresponding to the long spacing by using the PPG signal corresponding to the short spacing, and then determine the physiological parameter value by using a processed PPG signal corresponding to the long spacing.

It can be learned from steps S101 and S102 that the electronic device 100 may calculate the physiological parameter value by using a plurality of PPG signals. Compared with using only one PPG signal, using the plurality of PPG signals can exclude or mitigate impact of the signal corresponding to the superficial tissue on calculation of the physiological parameter value, to improve accuracy of physiological parameter measurement.

The following describes in detail a detailed process in which the electronic device 100 determines the physiological parameter value based on the n PPG signals by using an example in which the physiological parameter measured by the electronic device 100 is blood oxygen.

Blood oxygen is a percentage of an oxygen-bound oxyhemoglobin ($HbO_2$) capacity in a total oxygen-binding hemoglobin (Hb) capacity in blood, and may be specifically represented by using the following Formula 1:

$$\mathrm{SpO_2} = \frac{\text{oxyhemoglobin}}{\text{oxyhemoglobin+deoxyhemoglobin}} * 100\% \qquad \text{Formula 1}$$

Because oxyhemoglobin and deoxyhemoglobin have different absorption rates for light sources with different wavelengths, the electronic device 100 may calculate a ratio between absorption rates of light with different wavelengths, that is, a ratio value R, by using components of different light in a collected PPG signal, and then calculate a blood oxygen value of the user by using ratio values R correspondingly calculated by using different PPG signals.

**FIG. 5 is a diagram of a PPG signal according to an embodiment of this application.**

As shown in FIG. 5, the PPG signal may be decomposed into an AC component and a DC component. The AC component represents an alternating current part in the PPG signal, which has a same frequency change as a pulse, and is for determining a blood flow. The DC component represents a direct current part in the PPG signal, and represents a skin tissue and an average blood volume.

For example, if the electronic device 100 measures blood oxygen by using red light and infrared light as a light source, a PPG signal collected by the electronic device 100 may include two signals: a PPG signal corresponding to the red light and a PPG signal corresponding to the infrared light. In this case, a ratio value R may be calculated by using the following Formula 2:

$$\mathrm{R} = \frac{AC_{red}/DC_{red}}{AC_{IR}/DC_{IR}} \qquad \text{Formula 2}$$

$AC_{red}$ represents an AC component in the PPG signal corresponding to the red light, $DC_{red}$ represents a DC component in the PPG signal corresponding to the red light, $AC_{IR}$ represents an AC component in the PPG signal corresponding to the infrared light, and $DC_{IR}$ represents a DC component in the PPG signal corresponding to the infrared light.

For example, for a PPG signal corresponding to light with any wavelength, an AC component may be obtained by filtering the PPG signal by using a filter with a frequency of 0.5 to 5 Hz, and a DC component may be obtained by filtering the PPG signal by using a low-pass filter with a frequency of less than 0.5 Hz. Then, AC components and DC components that are needed in Formula 2 may be correspondingly obtained based on a peak position and a trough position in the AC component.

If the electronic device 100 calculates the blood oxygen value by using only one PPG signal, it is learned, according to a Beer-Lambert law, that the blood oxygen value $SpO_2$ is in a linear relationship with the ratio value R. Therefore, a mathematical relationship between the blood oxygen value $SpO_2$ and the ratio value R may be represented by using the following Formula 3:

$$\mathrm{SpO_2 = A - B * R} \qquad \text{Formula 3}$$

$SpO_2$ represents the blood oxygen value, R represents the ratio value, A and B are parameters. A and B may be obtained by fitting oxygen reduction experiment data.

In this embodiment of this application, when the electronic device 100 calculates the blood oxygen value by using the plurality of PPG signals, the electronic device 100 may construct a multi-spacing blood oxygen model shown in the following Formula 4:

$$SpO_2 = a_1 * R1 + a_2 * R2 + \ldots a_n * Rn + a_{n+1} \qquad \text{Formula 4}$$

$a_1, a_2, \ldots, a_n$, and $a_{n+1}$ may be parameters in the multi-spacing blood oxygen model, R1, R2, ..., and Rn represent n ratio values calculated by the electronic device 100 by using the PPG signals collected by the PPG assembly, and $SpO_2$ represents the blood oxygen value.

For example, the multi-spacing blood oxygen model may be constructed by using a method like multivariate linear fitting, a neural network, or XGBoost. The parameters $a_1, a_2, \ldots, a_n$, and $a_{n+1}$ in the multi-spacing blood oxygen model may be determined by using a plurality of data sets. One data set includes a blood oxygen value of a testee and k ratio values R calculated by using PPG signals collected by k ($k \geq 1$) detection modules from the measured person.

The following describes in detail, in FIG. 6, a process in which the electronic device 100 determines the blood oxygen value by using the n PPG signals.

**FIG. 6 is a detailed flowchart of a method for determining a blood oxygen value by using n PPG signals according to an embodiment of this application.**

As shown in FIG. 6, step S102 may be described in detail as the following steps.

**S201:** The electronic device 100 calculates one ratio value R for each PPG signal in the n PPG signals, where the ratio value R is for reflecting a ratio between absorption rates of light with different wavelengths in the PPG signal.

For each PPG signal in the n PPG signals, the electronic device 100 may calculate the ratio value R by using the foregoing Formula 2.

**S202:** The electronic device 100 determines the blood oxygen value based on n ratio values R corresponding to the n PPG signals.

For example, the electronic device 100 may input the n ratio values R into the multi-spacing blood oxygen model, to obtain the blood oxygen value. The multi-spacing blood oxygen model is configured to represent a mathematical relationship between a plurality of ratio values and blood oxygen. For the multi-spacing blood oxygen model, refer to the foregoing Formula 4.

For example, the multi-spacing blood oxygen model may be preset in the electronic device 100 by a developer in advance.

For example, assuming that n is 2, the n signals collected by the electronic device 100 may include a first PPG signal and a second PPG signal. The first PPG signal may be collected by a detection module with a long spacing, and the second PPG signal may be collected by a detection module with a short spacing. The electronic device 100 may calculate two ratio values: R1 and R2.

In this case, the electronic device 100 may input the ratio value R1 and the ratio value R2 into the following Formula 3, to calculate the blood oxygen value of the user:

$$SpO_2 = a * R1 + b * R2 + c \qquad \text{Formula 5}$$

a, b, and c may be parameters in the multi-spacing blood oxygen model. The multi-spacing blood oxygen model may be obtained through training by using PPG signals collected from the measured person by two detection modules with different spacings when blood oxygen of the measured person is known.

It may be understood that, in the foregoing Formula 4 and Formula 5, the multi-spacing blood oxygen model is constructed on the premise that the blood oxygen value is in a linear relationship with a plurality of ratio values R. In another embodiment of this application, the blood oxygen value and the ratio value R may alternatively be in another mathematical relationship. In this case, a mathematical formula of the multi-spacing blood oxygen model is not limited to the foregoing Formula 4 or Formula 5. For example, Formula 5 may alternatively be transformed into: $SpO_2 = a * R1^2 + b * R2 + c$. In other words, the foregoing Formula 4 or Formula 5 is not used to limit the multi-spacing blood oxygen model in this embodiment of this application.

The following describes, with reference to FIG. 7 to FIG. 12, a possible structure of the PPG assembly in embodiments of this application.

**FIG. 7 is a diagram of a structure of a PPG assembly according to an embodiment of this application.**

As shown in FIG. 7, the PPG assembly in the electronic device 100 may include one photoelectric detector PD and two light sources: an LED 1 and an LED 2. The LED 1 and the PD may form one detection module, the LED 2 and the PD may form one detection module, and a spacing between the LED 1 and the PD is less than a spacing between the LED 2 and the PD.

For example, when the electronic device 100 collects PPG signals, the electronic device 100 may first control the LED 1 to emit a light ray, and collect a light ray reflected from skin via the PD, to obtain a PPG signal corresponding to the spacing between the LED 1 and the PD; and then, the electronic device 100 may control the LED 2 to emit a light ray, and collect a light ray reflected from the skin via the PD, to obtain a PPG signal corresponding to the spacing between the LED 2 and the PD. In this way, the electronic device 100 may obtain PPG signals collected by two detection modules at different spacings, to measure the physiological parameter of the user by using the two PPG signals.

For example, if the physiological parameter is blood oxygen, after obtaining the two PPG signals, the electronic device 100 may respectively calculate ratio values R1 and R2 by using the two PPG signals, and then calculate the blood oxygen value of the user by using the foregoing Formula 5.

Based on FIG. 7, further, the PPG assembly may include two or more light sources. **For example, FIG. 8 is a diagram of another structure of a PPG assembly according to an embodiment of this application.**

As shown in FIG. 8, the PPG assembly in the electronic device 100 may include one photoelectric detector PD and n (n≥2) light sources: an LED 1, an LED 2, ..., and an LED n. Each of the n light sources may form one detection module with the photoelectric detector PD, and spacings of any two detection modules may be different.

Similar to related descriptions in FIG. 7, when the electronic device 100 collects PPG signals, the electronic device 100 may respectively control different light sources to emit light rays at different time points, so that the electronic device 100 may collect PPG signals at different time points via the PD, to obtain n PPG signals. In this way, the electronic device 100 may obtain PPG signals collected by n detection modules at different spacings, to measure the physiological parameter of the user by using the n PPG signals.

For example, if the physiological parameter is blood oxygen, after obtaining the n PPG signals, the electronic device 100 may respectively calculate ratio values R1, R2, ..., and Rn by using the n PPG signals, and then calculate the blood oxygen value of the user by using the foregoing Formula 4.

In addition, a plurality of detection modules included in the PPG assembly may include detection modules with a same spacing in addition to detection modules with different spacings.

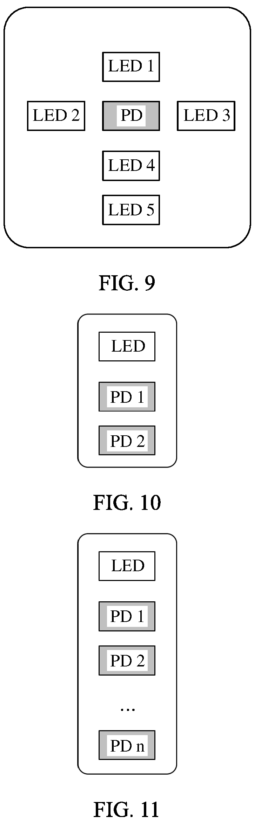

**For example, FIG. 9 is a diagram of another structure of a PPG assembly according to an embodiment of this application.**

As shown in FIG. 9, the PPG assembly in the electronic device 100 may include one photoelectric detector PD and five light sources: an LED 1, an LED 2, an LED 3, an LED 4, and an LED 5. Each of the five light sources may form one detection module with the photoelectric detector PD. In addition, spacings between the light sources LED 1, LED 2, LED 3, and LED 4 and the photoelectric detector PD are the same, and a spacing between the light source LED 5 and the photoelectric detector PD is greater than a spacing between any one of the light sources LED 1, LED 2, LED 3, and LED 4 and the photoelectric detector PD.

Similar to related descriptions in FIG. 7, when the electronic device 100 collects PPG signals, the electronic device 100 may respectively control different light sources to emit light rays at different time points, so that the electronic device 100 may collect PPG signals at different time points via the PD, to obtain five PPG signals. In this way, the electronic device 100 may obtain PPG signals collected by five detection modules at a plurality of spacings including a same spacing and different spacings, to measure the physiological parameter of the user by using the five PPG signals.

For example, if the physiological parameter is blood oxygen, after obtaining the five PPG signals, the electronic device 100 may respectively calculate ratio values R1, R2, ..., and R5 by using the five PPG signals, and then calculate the blood oxygen value of the user by using the foregoing Formula 4.

**FIG. 10 is a diagram of another structure of a PPG assembly according to an embodiment of this application.**

As shown in FIG. 10, the PPG assembly in the electronic device 100 may include one light source LED and two photoelectric detectors: a PD 1 and a PD 2. The PD 1 and the LED may form one detection module, the PD 2 and the LED may form one detection module, and a spacing between the PD 1 and the LED is less than a spacing between the PD 2 and the LED.

For example, when the electronic device 100 collects PPG signals, the electronic device 100 may control the LED to emit a light ray, and respectively collect light rays reflected from skin via the PD 1 and the PD 2, to obtain a PPG signal corresponding to the spacing between the LED and the PD 1 and a PPG signal corresponding to the spacing between the LED and the PD 2. In this way, the electronic device 100 may measure the physiological parameter of the user by using two PPG signals collected at different spacings.

For example, if the physiological parameter is blood oxygen, after obtaining the two PPG signals, the electronic device 100 may respectively calculate ratio values R1 and R2 by using the two PPG signals, and then calculate the blood oxygen value of the user by using the foregoing Formula 5.

Based on FIG. 10, further, the PPG assembly may include two or more photoelectric detectors. **For example, FIG. 11 is a diagram of another structure of a PPG assembly according to an embodiment of this application.**

As shown in FIG. 11, the PPG assembly in the electronic device 100 may include one light source LED and n (n≥2) photoelectric detectors: a PD 1, a PD 2, ..., and a PD n. Each of the n photoelectric detectors may form one detection module with the light source LED, and spacings of any two detection modules may be different.

Similar to related descriptions in FIG. 10, when the electronic device 100 collects PPG signals, the electronic device 100 may control the LED to emit a light ray, and respectively collect light rays reflected from skin via different PDs, to obtain PPG signals corresponding to spacings between the LED and the different PDs. In this way, the electronic device 100 may measure the physiological parameter of the user by using n PPG signals collected at different spacings.

For example, if the physiological parameter is blood oxygen, after obtaining the n PPG signals, the electronic device 100 may respectively calculate ratio values R1, R2, ..., and Rn by using the n PPG signals, and then calculate the blood oxygen value of the user by using the foregoing Formula 4.

In addition, a plurality of detection modules included in the PPG assembly may include detection modules with a same spacing in addition to detection modules with different spacings.

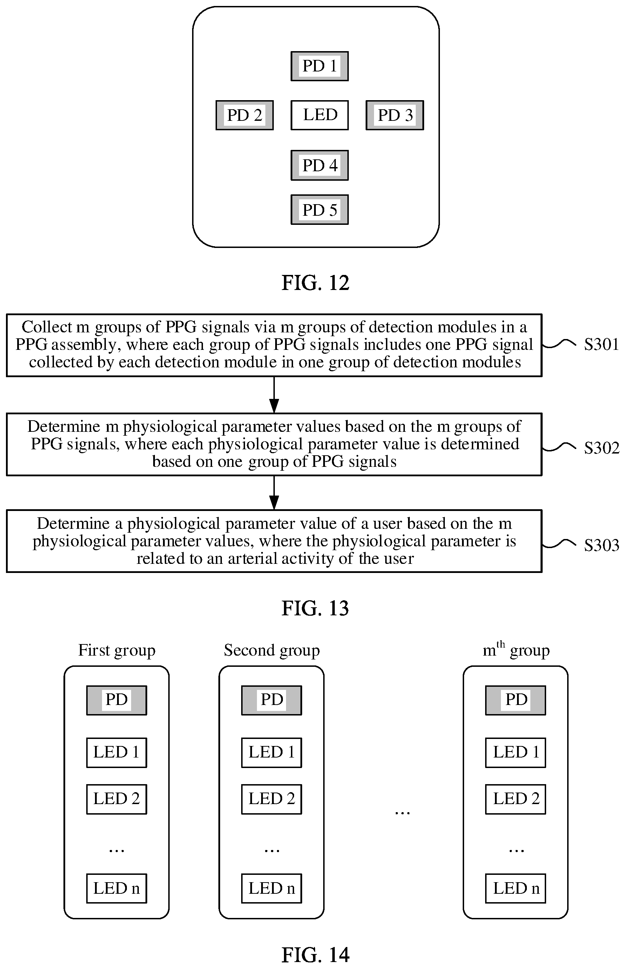

**For example, FIG. 12 is a diagram of another structure of a PPG assembly according to an embodiment of this application.**

As shown in FIG. 12, the PPG assembly in the electronic device 100 may include one light source LED and five photoelectric detectors: a PD 1, a PD 2, a PD 3, a PD 4, and a PD 5. Each of the five photoelectric detectors may form one detection module with the light source LED. In addition, spacings between the photoelectric detectors PD 1, PD 2, PD 3, and PD 4 and the light source LED are the same, and a spacing between the photoelectric detector PD 5 and the light source LED is greater than a spacing between any one of the photoelectric detectors PD 1, PD 2, PD 3, and PD 4 and the photoelectric detector PD.

Similar to related descriptions in FIG. 10, when the electronic device 100 collects PPG signals, the electronic device 100 may control the LED to emit a light ray, and respectively collect light rays reflected from skin via different PDs, to obtain PPG signals corresponding to spacings between the LED and the different PDs. In this way, the electronic device 100 may measure the physiological parameter of the user by using PPG signals collected by five detection modules at spacings including a same spacing and different spacings.

For example, if the physiological parameter is blood oxygen, after obtaining the five PPG signals, the electronic device 100 may respectively calculate ratio values R1, R2, ..., and R5 by using the five PPG signals, and then calculate the blood oxygen value of the user by using the foregoing Formula 4.

It may be understood that, an example in which the PPG assembly includes only one photoelectric detector is used in FIG. 7, FIG. 8, and FIG. 9, and an example in which the PPG assembly includes only one light source is used in FIG. 10, FIG. 11, and FIG. 12, to describe a possible structure of the PPG assembly. In another embodiment of this application, the PPG assembly may alternatively include more or fewer light sources and more or fewer photoelectric detectors. For example, the photoelectric detectors in the PPG assembly shown in FIG. 12 may include only the PD 5 and any two of the PD 1, the PD 2, the PD 3, and the PD 4.

It should be understood that, in embodiments of this application, quantities and positions of light sources and photoelectric detectors may not be limited provided that it is ensured that the light sources and the photoelectric detectors included in the PPG assembly can form at least detection modules with two spacings.

It can be learned from FIG. 7 to FIG. 12 that the PPG assembly in the electronic device 100 may include one or more light sources and a plurality of photoelectric detectors, or a plurality of light sources and one or more photoelectric detectors. In addition, the PPG assembly includes at least detection modules with two spacings. In this way, the electronic device 100 may accurately measure the physiological parameter of the user by using PPG signals collected by detection modules with different spacings.

In the method procedure shown in FIG. 4, the electronic device 100 may determine one physiological parameter value by using the n PPG signals collected by the n detection modules, and use the physiological parameter value as the physiological parameter value of the user.

Further, the n detection modules shown in FIG. 4 may be considered as one group of detection modules. If the PPG assembly in the electronic device 100 includes m (m≥2) groups of detection modules, and each group of detection modules may include one or more detection modules, for each group of detection modules, the electronic device 100 may determine one physiological parameter value by using one or more PPG signals in the group. In this way, for the m groups of detection modules, the electronic device 100 may obtain m physiological parameter values.

The m physiological parameter values may be considered as intermediate values, and the electronic device 100 may further perform specific fusion by using the m physiological parameter values to finally determine the physiological parameter value of the user, to improve stability and accuracy of physiological parameter measurement.

**For example, FIG. 13 is a schematic flowchart of another physiological parameter detection method according to an embodiment of this application.**

**S301:** An electronic device 100 collects m groups of PPG signals via m groups of detection modules in a PPG assembly, where each group of PPG signals includes one PPG signal collected by each detection module in one group of detection modules.

In other words, if one group of detection modules includes x detection modules, one group of PPG signals collected by the group of detection modules includes x PPG signals.

The electronic device 100 may be a wearable device worn by a user, for example, a watch, a band, or a ring.

The electronic device 100 may include the PPG assembly, and the PPG assembly may be the PPG assembly 193 in FIG. 3A.

The PPG assembly may include the m (m≥2) groups of detection modules. Each group of detection modules includes one or more detection modules. Each detection module may include one light source and one photoelectric detector. The photoelectric detector in the detection module may be configured to collect one PPG signal corresponding to the light source in the detection module.

It may be understood that different groups of detection modules may include different quantities of detection modules. For example, the PPG assembly may include a first group of detection modules and a second group of detection modules. The first group of detection modules may include one detection module, and the second group of detection modules may include two detection modules.

For example, the m groups of detection modules include one group of detection modules that includes at least detection modules with two spacings, and there may be one or more detection modules with each spacing. A spacing of a detection module is a distance between a light source and a photoelectric detector in the detection module. In this case, one group of PPG signals (for example, a first group of PPG signals) in the m groups of PPG signals collected by the electronic device 100 includes at least PPG signals collected by two detection modules with different spacings.

In this way, one group of PPG signals collected by the electronic device 100 may include at least two PPG signals that have different compositions. The composition may be proportions corresponding to tissues of different depths. In this way, when calculating one physiological parameter value by using one group of PPG signals, the electronic device 100 can eliminate or suppress, by using PPG signals corresponding to different spacings, an interference signal corresponding to a superficial tissue, so that the calculated physiological parameter value is more accurate.

It may be understood that quantities and layouts of light sources and photoelectric detectors in different groups of detection modules may be the same or different. For example, a light source and a photoelectric detector in the first group of detection modules may be the light sources and the photoelectric detector that are included in the PPG assembly shown in FIG. 7, and a light source and a photoelectric detector in the second group of light sources-photoelectric detectors may be the light source and the photoelectric detectors that are included in the PPG assembly shown in FIG. 12.

In some implementations, in the m groups of detection modules, a device in one group and a device in another group may be independent of each other, or a device may be shared by one group and another group. In this case, if the m groups of detection modules include two groups of detection modules that share a device, the two groups of detection modules may include at least a same light source and/or at least a same photoelectric detector. The device is shared by the different groups of detection modules, to help reduce costs and power consumption.

In addition, for example, after detecting a user operation of measuring a physiological parameter by the user, the electronic device 100 may trigger step S301 to be performed. Alternatively, when a preset condition is met, for example, preset time is reached, or it is detected that the user is in a still state, the electronic device 100 may trigger step S301 to be performed. An occasion at which the electronic device 100 triggers step S301 to be performed is not limited in this embodiment of this application. For specific related descriptions of triggering, by the electronic device 100, step S301 to be performed, refer to related content in step S101. Details are not described herein again.

**S302:** The electronic device 100 determines m physiological parameter values based on the m groups of PPG signals, where each physiological parameter value is determined based on one group of PPG signals.

For the m groups of PPG signals, the electronic device 100 may determine one physiological parameter value for each group of PPG signals, to obtain the m physiological parameter values.

If one group of PPG signals includes only one PPG signal, the electronic device 100 may determine one physiological parameter value by using the one PPG signal.

For example, if the physiological parameter is blood oxygen, the electronic device 100 may determine one physiological parameter value by using one PPG signal according to the foregoing Formula 2 and Formula 3.

It should be understood that a calculation manner in which the electronic device 100 calculates a physiological parameter value by using one PPG signal may be the same as a manner in which the electronic device 100 calculates the physiological parameter of the user by using a PPG signal in a conventional technology. Details are not described herein again.

If one group of PPG signals includes a plurality of PPG signals, the electronic device 100 may determine one physiological parameter value by using the plurality of PPG signals.

During specific implementation, the electronic device 100 may eliminate, by using a PPG signal that corresponds to a short spacing and that is included in the plurality of PPG signals, an interference signal that is from the superficial tissue and that is included in a PPG signal corresponding to a long spacing, and then determine the physiological parameter value by using a processed PPG signal corresponding to the long spacing.

For example, if the physiological parameter value is blood oxygen, the electronic device 100 may determine one physiological parameter value by using the plurality of PPG signals according to the foregoing Formula 2 and Formula 4.

If one group of detection modules includes a plurality of detection modules, for details about a process in which the plurality of detection modules collect PPG signals, a process in which a physiological parameter value is determined by using a plurality of PPG signals, and a physical layout of the plurality of detection modules, refer to content in FIG. 4 to FIG. 12. Details are not described herein again.

It should be noted that when the electronic device 100 collects PPG signals via the m groups of detection modules, the electronic device 100 may simultaneously start the m groups of detection modules to collect the m groups of PPG signals, or may respectively start different groups of detection modules at different time points to collect the PPG signals. This may be specifically determined by a layout of a light source and a photoelectric detector in each group of detection modules in the m groups of detection modules. For details about how the electronic device 100 starts the m groups of detection modules, refer to related content in PPG assemblies enumerated in FIG. 14 to FIG. 20 subsequently. Details are not described herein.

It may be understood that an execution sequence of step S301 and step S302 is not limited in this embodiment of this application. The electronic device 100 may first perform step S301, and then perform step S302. In other words, the electronic device 100 may calculate a physiological parameter value corresponding to each group of PPG signals after collecting the m groups of PPG signals. Alternatively, the electronic device 100 may simultaneously perform steps S301 and S302. In other words, the electronic device 100 may calculate a physiological parameter value when collecting a PPG signal. In this case, it only needs to ensure that one group of detection modules thereof completes collection of one group of PPG signals, and then, calculation of a physiological parameter value corresponding to the group of detection modules may be started.

S303: The electronic device 100 determines a physiological parameter value of the user based on the m physiological parameter values, where the physiological parameter is related to an arterial activity of the user.

For example, the electronic device 100 may use a fusion algorithm for the m physiological parameter values, to calculate the physiological parameter value of the user. The fusion algorithm may be taking a largest value, a smallest value, an average value, a median, a mode, or the like.

In other words, the physiological parameter value of the user may be a largest value, a smallest value, an average value, a median, or a mode of the m physiological parameter values.

An example in which the physiological parameter is blood oxygen is used. It is assumed that the electronic device 100 calculates, by using the m groups of detection modules, m blood oxygen values: $SpO_2\_1$, $SpO_2\_2$, ..., and $SpO_2\_m$. One blood oxygen value is calculated based on one group of PPG signals collected by one group of detection modules in the m groups of detection modules. If the fusion algorithm takes an average value, a blood oxygen value of the user= $(SpO_2\_1+SpO_2\_2+...+SpO_2\_m)/m$.

This is because when the user actually wears the electronic device 100, signal quality of a PPG signal collected at some positions at which the PPG assembly is located may be poor due to various factors. For example, because the user wears the electronic device 100 improperly, skin may not be in close contact with a partial area in the PPG assembly, and signal quality of a PPG signal collected by a detection module in the area is poor. In this case, if the physiological parameter of the user is directly calculated by using the PPG signal with poor signal quality, a calculation result may be incorrect, and evaluation on a body status of the user is affected.

Therefore, a plurality of groups of PPG signals are collected by using a plurality of groups of detection modules, and then a final physiological parameter value of the user is comprehensively calculated by using a plurality of physiological parameter values calculated based on the plurality of groups of PPG signals. This can mitigate impact of a wearing position of the PPG assembly on accuracy of physiological parameter measurement as much as possible, to improve accuracy and stability of physiological parameter measurement.

The following describes, with reference to FIG. 14 to FIG. 20, a possible structure of the PPG assembly in embodiments of this application.

**FIG. 14 is a diagram of another structure of a PPG assembly according to an embodiment of this application.**

As shown in FIG. 14, the PPG assembly in the electronic device 100 may include m (m≥2) groups of detection modules: a first group of detection modules, a second group of detection modules, ..., and an $m^{th}$ group of detection modules. One group of detection modules may include one photoelectric detector PD and n (n≥2) light sources: an LED 1, an LED 2, ..., and an LED n. Each of the n light sources may form one detection module with the photoelectric detector PD, and spacings of any two detection modules may be different.

In this case, when collecting PPG signals, the electronic device 100 may first start the first group of detection modules and collect n PPG signals via the first group of detection modules, then start the second group of detection modules and collect n PPG signals via the second group of detection modules, and so on, and finally start the $m^{th}$ group of detection modules and collect n PPG signals via the $m^{th}$ group of detection modules. When the electronic device 100 measures a physiological parameter, the electronic device 100 may calculate a first physiological parameter value based on the n PPG signals collected by the first group of detection modules, calculate a second physiological parameter value based on the n PPG signals collected by the second group of light sources-photoelectric detectors, ..., and calculate an $m^{th}$ physiological parameter value based on the n PPG signals collected by the $m^{th}$ group of light sources-photoelectric detectors. Finally, the fusion algorithm is used for m physiological parameter values, to calculate the final physiological parameter value of the user.

For a specific working process of the light sources and the photoelectric detector in each group of detection modules, refer to related content in FIG. 8. Details are not described herein again.

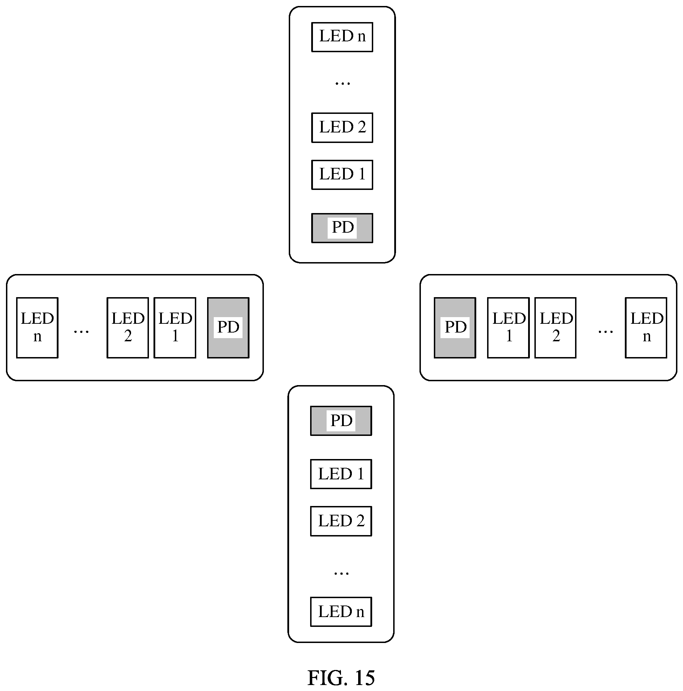

It may be understood that, in the PPG assembly shown in FIG. 14, the groups of detection modules are horizontally arranged in sequence. In another embodiment of this application, the groups of detection modules may alternatively be arranged in another manner. For example, assuming that m is equal to 4, the four groups of detection modules may be arranged in a placement manner shown in FIG. 15. In addition, when the PPG assembly shown in FIG. 14 collects PPG signals, the groups of detection modules may be simultaneously started to collect the PPG signals. An execution sequence of a plurality of groups of detection modules in the PPG assembly is not limited in this embodiment of this application. Subsequent PPG assemblies shown in FIG. 15 to FIG. 20 are similar, and details are not described below again.

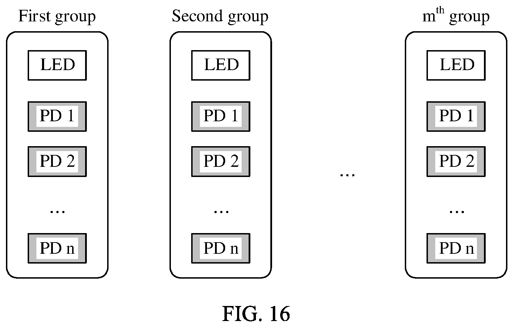

**FIG. 16 is a diagram of another structure of a PPG assembly according to an embodiment of this application.**

As shown in FIG. 16, the PPG assembly in the electronic device 100 may include m (m$\geq$2) groups of detection modules: a first group of detection modules, a second group of detection modules, ..., and an m$^{th}$ group of detection modules. One group of detection modules may include one light source LED and n (n$\geq$2) photoelectric detectors: a PD 1, a PD 2, ..., and a PD n. Each of the n photoelectric detectors may form one detection module with the light source LED, and spacings of any two detection modules may be different.

For specific descriptions of measuring physiological parameters by the electronic device 100 via the m groups of detection modules in the PPG assembly shown in FIG. 16, refer to related descriptions in FIG. 14. For a specific working process of the light source and the photoelectric detectors in each group of detection modules, refer to related content in FIG. 11. Details are not described herein again.

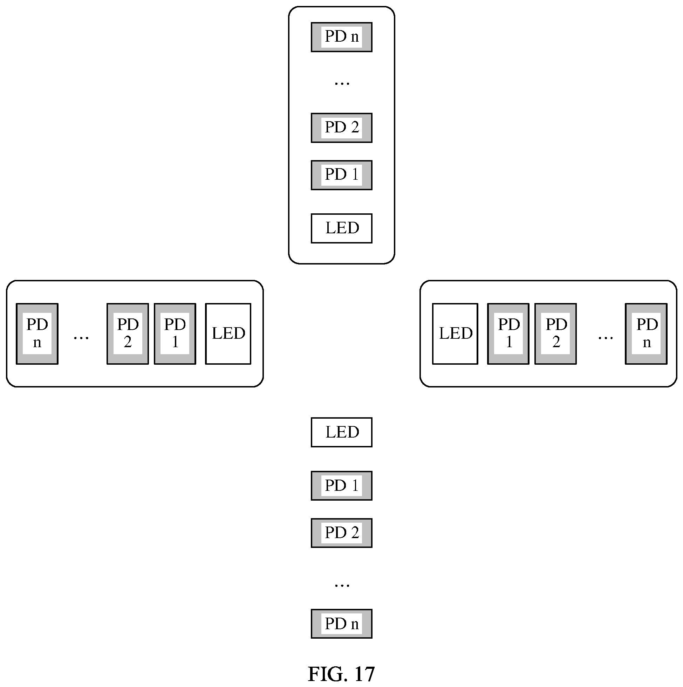

It may be understood that, in the PPG assembly shown in FIG. 16, the groups of detection modules are horizontally arranged in sequence. In another embodiment of this application, the groups of detection modules may alternatively be arranged in another manner. For example, assuming that m is equal to 4, the four groups of detection modules may be arranged in a placement manner shown in FIG. 17. For a specific working process of the light source and the photoelectric detectors in any group of detection modules in the PPG assembly shown in FIG. 17, refer to descriptions in FIG. 11. Details are not described herein again.

It can be learned from the PPG assemblies shown in FIG. 14 to FIG. 17 that when the PPG assembly includes a plurality of groups of light sources-photoelectric detectors, devices included in each group of light sources-photoelectric detectors are independent of each other.

In some implementations, when the PPG assembly includes a plurality of groups of detection modules, a device may be shared by one group and another group, to help reduce costs and power consumption.

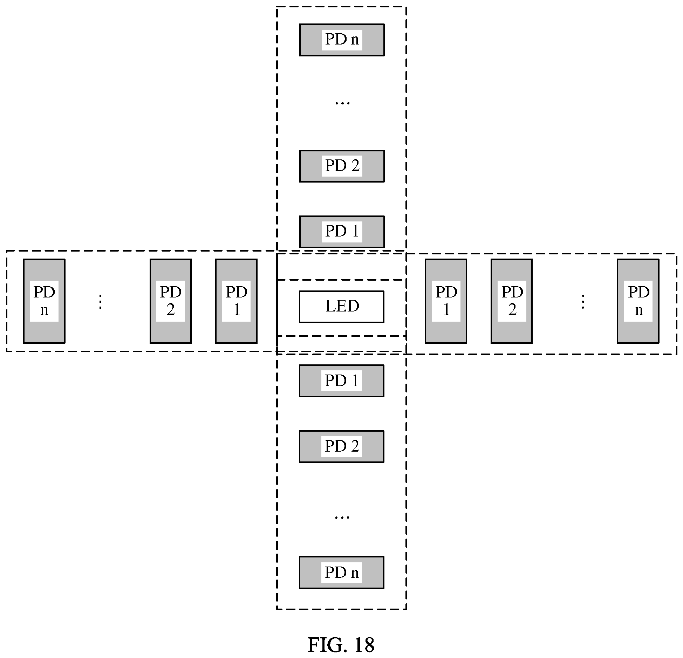

**FIG. 18 is a diagram of another structure of a PPG assembly according to an embodiment of this application.**

As shown in FIG. 18, the PPG assembly in the electronic device 100 may include four groups of detection modules. Each group of detection modules includes one light source LED and n photoelectric detectors, and each of the n photoelectric detectors may form one detection module with the light source LED. It can be learned that the four groups of detection modules share one light source.

In this way, when the electronic device 100 measures a physiological parameter, the electronic device 100 may start a light source LED to emit a light ray; then collect, via photoelectric detectors in each group of light sources-photoelectric detectors, light rays reflected from skin, to obtain four groups of n PPG signals; then calculate four physiological parameter values by using the four groups of n PPG signals; and finally use the fusion algorithm, to calculate the final physiological parameter value of the user.

In the PPG assembly shown in FIG. 18, the groups of detection modules may be simultaneously started, to quickly obtain a PPG signal corresponding to each group of detection modules, to accelerate a speed of measuring the physiological parameter by the electronic device 100.

For a specific working process of the light source and the photoelectric detectors in any group of detection modules in the PPG assembly shown in FIG. 18, refer to descriptions in FIG. 11. Details are not described herein again.

**FIG. 19 is a diagram of another structure of a PPG assembly according to an embodiment of this application.**

As shown in FIG. 19, the PPG assembly in the electronic device 100 may include four light sources: an LED 1, an LED 2, an LED 3, and an LED 4, and three photoelectric detectors: a PD 1, a PD 2, and a PD 3.

For example, the plurality of light sources and the plurality of photoelectric detectors may form three groups of detection modules. A first group of detection modules may include one detection module including the LED 4 and the PD 1, and one detection module including the LED 2 and the PD 1. A second group of detection modules may include one detection module including the LED 1 and the PD 2, and one detection module including the LED 2 and the PD 2. A third group of detection modules may include one detection module including the LED 3 and the PD 3, and one detection module including the LED 2 and the PD 3.

It can be learned that in the PPG assembly shown in FIG. 19, the LED 2 is a device shared by the groups of detection modules.

For example, when the electronic device 100 collects PPG signals, the electronic device 100 may first start the LED 2, and collect PPG signals corresponding to the LED 2 via the PD 1, the PD 2, and the PD 3; and then respectively start the LED 1, the LED 3, and the LED 4 at different time points, collect a PPG signal corresponding to the LED 1 via the PD 2, collect a PPG signal corresponding to the LED 3 via the PD 3, and collect a PPG signal corresponding to the LED 4 via the PD 1. In this way, the electronic device 100 may collect two PPG signals corresponding to each group of detection modules; then calculate a physiological parameter value corresponding to each group of detection modules by using the two PPG signals of each group of detection modules; and finally calculate the final physiological parameter value of the user based on the fusion algorithm and three physiological parameter values corresponding to the three groups of detection modules.

It may be understood that, in addition to the combination manner shown in FIG. 19 for obtaining the groups of detection modules through division, another division manner may be used to set detection modules included in each group of detection modules. For example, the second group of detection modules may further include one detection module including the LED 1 and the PD 1.

**FIG. 20 is a diagram of another structure of a PPG assembly according to an embodiment of this application.**

As shown in FIG. 20, the PPG assembly in the electronic device 100 may include six light sources: an LED 1, an LED 2, an LED 3, an LED 4, an LED 5, and an LED 6, and six photoelectric detectors: a PD 1, a PD 2, a PD 3, a PD 4, a PD 5, and a PD 6.

For example, the plurality of light sources and the plurality of photoelectric detectors may form a plurality of groups of detection modules, and each group of detection modules may include at least two detection modules. A dashed line area shown in FIG. 20 shows an example of light sources and a photoelectric detector included in one group of detection modules. As shown in FIG. 20, the group of detection modules includes two light sources: an LED 1 and an LED 5, and one photoelectric detector PD 5. In addition, the light source LED 1 and the photoelectric detector PD 5 form one detection module, and the light source LED 5 and the photoelectric detector PD 5 form one detection module.

It may be understood that there may be a plurality of possibilities for a quantity of groups of detection modules included in the PPG assembly shown in FIG. 20, and quantities of light sources and photoelectric detectors included in each group of detection modules. The quantity of groups of detection modules included in the PPG assembly shown in FIG. 20, and the quantities of light sources and photoelectric detectors included in each group of detection modules are not limited in this embodiment of this application.

In addition, it should be noted that FIG. 14 to FIG. 20 are merely examples, and do not constitute a limitation on embodiments of this application.

In addition, it should be noted that, in addition to the physiological parameter detection method shown in FIG. 13, the PPG assemblies shown in FIG. 14 to FIG. 20 may also be used in the physiological parameter detection method shown in FIG. 4. In other words, in the PPG assemblies shown in FIG. 14 to FIG. 20, the electronic device 100 may use a physiological parameter value correspondingly calculated by only one group of detection modules as the physiological parameter value of the user. Alternatively, in the PPG assemblies shown in FIG. 14 to FIG. 20, a plurality of groups of light sources-photoelectric detectors may be combined into one group. In this way, after obtaining a PPG signal collected by each detection module in the PPG assembly, the electronic device 100 may directly calculate the physiological parameter of the user by using all collected PPG signals.

In addition, it should be noted that, in addition to the physiological parameter detection method shown in FIG. 4, FIG. 8, FIG. 9, FIG. 11, and FIG. 12 may also be applied to the physiological parameter detection method shown in FIG. 13.

The PPG assembly shown in FIG. 9 is used as an example. The PPG assembly may be divided into four groups of detection modules. First three groups of detection modules may include an LED 1 and a PD, an LED 2 and the PD, and an LED 3 and the PD respectively. A last group of detection modules may include an LED 4, an LED 5, and the PD.

In other words, the first three groups of light sources-photoelectric detectors each may include only one detection module, and the last group of detection modules may include two detection modules. The electronic device 100 may collect one PPG signal via the LED 1 and the PD, and calculate one physiological parameter value by using the PPG signal. Similarly, the electronic device 100 collects one PPG signal via the LED 2 and the PD, and then calculates one physiological parameter value by using the PPG signal; collects one PPG signal via the LED 3 and the PD, and then calculates one physiological parameter value by using the PPG signal; and collects two PPG signals via the LED 4, the LED 5, and the PD, and then calculates one physiological parameter value by using the two PPG signals. Finally, the physiological parameter value of the user is calculated based on the fusion algorithm and the four physiological parameter values.

Further, power consumption of the electronic device 100 is high when a plurality of detection modules are started at a time to measure the physiological parameter of the user. Therefore, when starting to measure the physiological parameter of the user, the electronic device 100 may start only one detection module to measure the physiological parameter of the user. When determining that a measurement effect is poor, the electronic device 100 uses the physiological parameter detection method provided in embodiments of this application, to measure the physiological parameter of the user via a plurality of detection modules or a plurality of groups of detection modules, thereby effectively reducing power consumption of the electronic device 100.

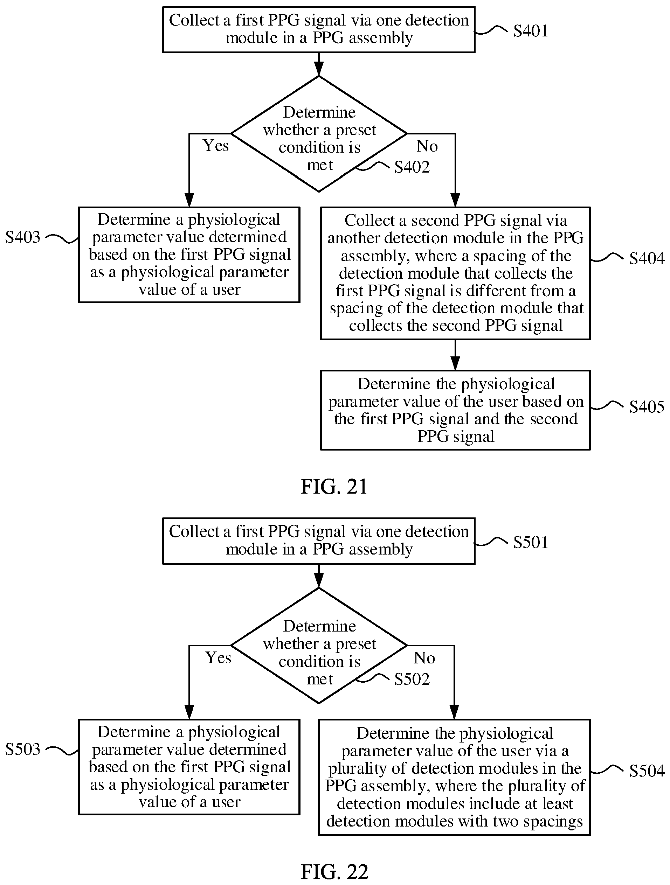

**FIG. 21 is a schematic flowchart of another physiological parameter detection method according to an embodiment of this application.**

**S401:** An electronic device 100 collects a first PPG signal via one detection module in a PPG assembly.

The electronic device 100 may include the PPG assembly. The PPG assembly may include at least two detection modules, and spacings of the two detection modules are different. One detection module may include one light source and one photoelectric detector. A spacing of a detection module may be a distance between a light source and a photoelectric detector in the detection module.

In addition, for example, after detecting a user operation of measuring a physiological parameter by a user, the electronic device 100 may trigger step S401 to be performed. Alternatively, when a preset condition is met, for example, preset time is reached, or it is detected that the user is in a still state, the electronic device 100 may trigger step S401 to be performed. An occasion at which the electronic device 100 triggers step S401 to be performed is not limited in this embodiment of this application. For specific related descriptions of triggering, by the electronic device 100, step S401 to be performed, refer to related content in step S101. Details are not described herein again.

For a specific implementation process in which the electronic device 100 collects the first PPG signal via one detection module, refer to a detailed process in which the electronic device 100 collects one PPG signal via one detection module in step S101. Details are not described herein again.

**S402:** The electronic device 100 determines whether the preset condition is met.

If the preset condition is met, step S403 is performed, to be specific, the electronic device 100 determines that a physiological parameter value determined based on the first PPG signal is a physiological parameter value of the user; or if the preset condition is not met, step S404 is performed, to be specific, the electronic device 100 continues to collect a PPG signal via another detection module in the PPG assembly, and determines the physiological parameter value of the user based on the continuously collected PPG signal.

For example, the preset condition may include any one or more of the following:

(1) The physiological parameter value reflected by the first PPG signal is normal.

That the physiological parameter value reflected by the first PPG signal is normal may mean that the physiological parameter value reflected by the first PPG signal is in a normal range.

For example, if the physiological parameter detected by the electronic device 100 is blood oxygen, the preset condition may be that a blood oxygen value is greater than or equal to 95%.

In other words, if the electronic device 100 detects, via one detection module, that the physiological parameter value of the user is in the normal range, it may be considered that the physiological indicator value detected by the current detection module can be for reflecting a current physiological parameter status of the user. If the electronic device 100 detects, via one detection module, that the physiological parameter value of the user is not in the normal range, it is considered that false detection may exist, and PPG signals collected by a plurality of pairs of detection modules may be further used to implement more accurate physiological parameter measurement.

(2) A user operation of agreeing, by the user, to use the physiological parameter value reflected by the first PPG signal as the physiological parameter value of the user is detected.

For example, after determining the physiological parameter value reflected by the first PPG signal, the electronic device 100 may display the physiological parameter value, and prompt the user whether to use the physiological parameter value as the physiological parameter value of the user. If the electronic device 100 detects the user operation of agreeing, by the user, to use the physiological parameter value as the physiological parameter value of the user, the electronic device 100 may perform step S403; or if the electronic device 100 does not detect the user operation of agreeing, by the user, to use the physiological parameter value as the physiological parameter value of the user, the electronic device 100 may perform step S404.

(3) A pulse reflected by the first PPG signal is complete.

If the electronic device 100 can clearly identify a pulse of the user based on the first PPG signal, it indicates that signal quality of the first PPG signal collected by the electronic device 100 is good. In this case, the first physiological parameter value determined by using the third PPG signal can be for reflecting a real status of the user. Therefore, the electronic device 100 may perform step S403.

If the electronic device 100 does not clearly identify the pulse of the user based on the first PPG signal, it indicates that the signal quality of the first PPG signal collected by the electronic device 100 is poor. In this case, the physiological parameter value determined by using the first PPG signal cannot be for reflecting the real status of the user. Therefore, the electronic device 100 may perform step S404.

It may be understood that the preset condition may alternatively include another case, and the preset condition shall fall within the protection scope of this application provided that the preset condition can reflect an effect of measuring the physiological parameter by using the first PPG signal. The preset condition is not limited in this embodiment of this application.

It should be noted that the physiological parameter value reflected by the first PPG signal may be calculated by the electronic device 100 based on the first PPG signal. The calculation method may be a method for determining a physiological parameter value based on one PPG signal in a conventional technology. This is not limited herein. For example, if the physiological parameter is blood oxygen, the electronic device 100 may calculate a ratio value R based on the first PPG signal, and then calculate the physiological parameter value by using the foregoing Formula 3.

S403: The electronic device 100 determines the physiological parameter value determined based on the first PPG signal as the physiological parameter value of the user.

If the electronic device 100 determines that the preset condition is met, the electronic device 100 may directly determine that a physiological parameter value determined by using a PPG signal collected by one detection module is the physiological parameter value of the user. In this way, steps in which the electronic device 100 starts a plurality of pairs of detection modules to detect the physiological parameter of the user can be simplified, and power consumption of the electronic device 100 can be reduced.

S404: The electronic device 100 collects a second PPG signal via another detection module in the PPG assembly, where a spacing of the detection module that collects the first PPG signal is different from a spacing of the detection module that collects the second PPG signal.

If the electronic device 100 determines that the preset condition is not met, the electronic device 100 may continue to start another detection module in the PPG assembly to continue to collect a PPG signal.

In addition, the spacing of the detection module started in step S404 is different from the spacing of the detection module started in step S401, so that proportions of signals corresponding to different tissue depths in the first PPG signal and the second PPG signal may be different.

It may be understood that, in addition to the detection modules in step S401 and step S402, the PPG assembly in the electronic device 100 may further include another detection module. A quantity of detection modules included in the PPG assembly in the electronic device 100 is not limited in this embodiment of this application.

S405: The electronic device 100 determines the physiological parameter value of the user based on the first PPG signal and the second PPG signal.

The electronic device 100 may eliminate or suppress, by using a PPG signal that corresponds to a short spacing and that is in the first PPG signal and the second PPG signal, a signal that corresponds to a superficial tissue and that is in a PPG signal corresponding to a long spacing, and then determine the physiological parameter value of the user by using a processed PPG signal corresponding to the long spacing.

For example, assuming that the physiological parameter is blood oxygen, the electronic device 100 may respectively determine ratio values R based on the first PPG signal and the second PPG signal, and then input the two ratio values R into a multi-spacing blood oxygen model, to obtain the physiological parameter value of the user. For specific related descriptions of the multi-spacing blood oxygen model, refer to related content of the foregoing Formula 5. Details are not described herein again.

It may be understood that, in steps S403 to S405, an example in which the electronic device 100 starts one detection module again when the preset condition is not met is used. In another embodiment of this application, when the electronic device 100 determines that the preset condition is not met, the electronic device 100 may start L (L≥1) detection modules. Spacings of the L detection modules may be the same, different, or partially the same.

In this way, when the electronic device 100 starts a detection module again, L PPG signals may be collected. In this case, the electronic device 100 may determine the physiological parameter value of the user by using the L PPG signals and the first PPG signal. For a detailed process in which the electronic device 100 determines the physiological parameter value of the user by using the L+1 PPG signals, refer to content in FIG. 4 to FIG. 6. Details are not described herein again.

Further, the L+1 PPG signals may alternatively be split into a plurality of groups of PPG signals. In this way, the electronic device 100 may calculate a plurality of physiological parameter values, and then determine the physiological parameter value of the user by using a fusion algorithm.

For a specific process in which the electronic device 100 determines the physiological parameter value of the user by using the plurality of groups of PPG signals, refer to content in FIG. 13. Details are not described herein again.

It can be learned from steps S401 to S405 that the electronic device 100 may split PPG signal collection operations performed by a plurality of detection modules. The electronic device 100 does not need to start the plurality of detection modules to collect PPG signals each time the physiological parameter is measured. Instead, the electronic device 100 preferentially starts one detection module, and measures the physiological parameter value of the user by using a PPG signal collected by the detection module. If a measurement effect is poor, the electronic device 100 starts a remaining detection module, and determines the physiological parameter value of the user by using PPG signals collected by all detection modules. In this way, power consumption of the electronic device 100 can be reduced as much as possible when accuracy of physiological parameter detection is ensured.

In steps S401 to S405, the electronic device 100 may first collect one PPG signal. If the electronic device 100 determines that the preset condition is not met, the electronic device 100 may collect one or more PPG signals again, and then determine the physiological parameter value of the user by using the one or more PPG signals collected again and the PPG signal first collected. In the following implementation, when determining that the preset condition is not met, the electronic device 100 may collect a plurality of PPG signals again, and determine the physiological parameter value of the user only by using the plurality of PPG signals collected again, instead of determining the physiological parameter value of the user with reference to the PPG signal first collected.

**FIG. 22 is a schematic flowchart of another physiological parameter detection method according to an embodiment of this application.**

**S501:** An electronic device 100 collects a first PPG signal via one detection module in a PPG assembly.

**S502:** The electronic device 100 determines whether a preset condition is met.

If the preset condition is met, step S503 is performed; or if the preset condition is not met, step S504 is performed.

**S503:** The electronic device 100 determines a physiological parameter value determined based on the first PPG signal as a physiological parameter value of a user.

If the electronic device 100 determines that the preset condition is met, the electronic device 100 may directly determine that a physiological parameter value determined by using a PPG signal collected by one detection module is the physiological parameter value of the user. In this way, steps in which the electronic device 100 starts a plurality of detection modules to detect the physiological parameter of the user can be simplified, and power consumption of the electronic device 100 can be reduced.

For specific descriptions of steps S501 to S503, refer to related content in steps S401 to S403. Details are not described herein again.

**S504:** The electronic device 100 determines the physiological parameter value of the user via a plurality of detection modules in the PPG assembly, where the plurality of detection modules include at least detection modules with two spacings.

If the electronic device 100 determines that the preset condition is not met, the electronic device 100 may start the plurality of detection modules in the PPG assembly, and determine the physiological parameter value of the user by using PPG signals collected by the plurality of detection modules.

The electronic device 100 may determine the physiological parameter value of the user via the plurality of detection modules in the following two manners:

(1) The electronic device 100 may obtain the PPG signals respectively collected by the plurality of detection modules, and then determine the physiological parameter value of the user based on the PPG signal collected by each detection module.

Specifically, the electronic device 100 may eliminate or suppress, by using a PPG signal that corresponds to a short spacing and that is in a plurality of collected PPG signals, an interference signal that is from the superficial tissue and that is included in a PPG signal corresponding to a long spacing, and then determine the physiological parameter value of the user by using a PPG signal obtained after the interference signal is eliminated or suppressed.

For a specific process in which the electronic device 100 determines the physiological parameter value of the user by using the plurality of PPG signals, refer to content in FIG. 4 to FIG. 6. Details are not described herein again.

(2) The plurality of detection modules may be divided into m groups of detection modules. Each group of detection modules may include one or more detection modules. The electronic device 100 may determine one physiological parameter value by using each group of detection modules, and then determine the physiological parameter value of the user based on a plurality of physiological parameter values.

The electronic device 100 may use a fusion algorithm for the plurality of physiological parameter values, and an obtained physiological parameter value is the physiological parameter value of the user.

For a specific description process in which the electronic device 100 determines the physiological parameter value of the user by using a plurality of groups of detection modules, refer to content in FIG. 13. Details are not described herein again.

It may be understood that one detection module in step S501 may be one of the plurality of detection modules in step S504, or may be different from any one of the plurality of detection modules in step S504. In addition, the plurality of detection modules in step S504 may be all or some of detection modules in the PPG assembly in the electronic device 100.

It can be learned from steps S501 to S504 that, when starting physiological parameter detection, the electronic device 100 may preferentially use only one detection module to measure a physiological parameter of the user, to reduce power consumption of the electronic device 100 as much as possible. If a measurement result is abnormal, the electronic device 100 starts a plurality of detection modules or a plurality of groups of detection modules to measure the physiological parameter of the user, to ensure accuracy of physiological parameter measurement as much as possible.

It should be noted that, in FIG. 21 and FIG. 22, the electronic device 100 preferentially calculates the physiological parameter by using a PPG signal collected by only one detection module, and then calculates the physiological parameter by using PPG signals collected by a plurality of detection modules. In another embodiment of this application, alternatively, the electronic device 100 may first start some detection modules in the PPG assembly, and measure the physiological parameter by using a plurality of PPG signals collected by the some detection modules; and start one or more detection modules in the PPG assembly again when a measurement effect is poor, and calculate the physiological parameter by using PPG signals collected by a plurality of detection modules. The detection module started again may be some or all of the detection modules in the PPG assembly. In other words, step S401 or step S501 may be replaced with that the electronic device 100 collects PPG signals via a plurality of detection modules in the PPG assembly, to obtain a plurality of PPG signals. Spacings of the plurality of detection modules may be the same or different. It should be understood that in the embodiments shown in FIG. 21 and FIG. 22, a quantity of detection modules first started by the electronic device 100 is not limited in embodiments of this application.

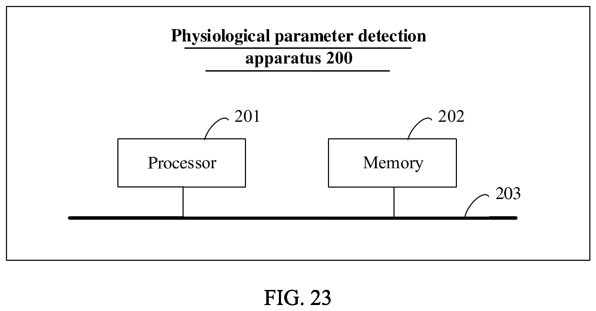

**FIG. 23 is a diagram of a structure of a physiological parameter detection apparatus 200 according to an embodiment of this application.**

As shown in FIG. 23, the physiological parameter detection apparatus 200 may include components such as a processor 201 and a memory 202. The components may be connected through a bus 203 or in another manner. In FIG. 23, a connection through the bus is used as an example. The bus 203 is configured to implement a connection and communication between the processor 201 and the memory 202.

In addition, the physiological parameter detection apparatus 200 may further include a PPG assembly. The PPG assembly may include n (n≥2) detection modules or m (m≥2) groups of detection modules. One group of detection modules includes one or more detection modules, and one detection module includes one light source and one photoelectric detector.

The processor 201 may include one or more processing units. The processor 201 may be configured to provide computing and control capabilities, and support running of the entire physiological parameter detection apparatus 200.

The memory 202 is configured to store various software programs and/or a plurality of groups of instructions. Specifically, the memory 202 may include a high-speed random access memory, or a nonvolatile memory, for example, one or more disk storage devices, a flash storage device, or another nonvolatile solid-state storage device.

In this embodiment of this application, the physiological parameter detection apparatus 200 may be the foregoing electronic device 100. The processor 201 may be configured to control the n detection modules in the PPG assembly to collect n PPG signals, and determine a physiological parameter value of a user based on the n PPG signals; or the processor 201 may be configured to control the m groups of detection modules in the PPG assembly to collect m groups of PPG signals, where one group of PPG signals includes one or more PPG signals, determine m physiological parameter values based on the m groups of PPG signals, and determine the physiological parameter value of the user based on the m physiological parameter values. The memory 202 may be configured to store the PPG signals collected by the PPG assembly and the calculated physiological parameter value.

It should be noted that the physiological parameter detection apparatus 200 shown in FIG. 23 is merely an implementation of this embodiment of this application. During actual application, the physiological parameter detection apparatus 200 may include more or fewer components than those shown in the figure, or combine some components, or have different component deployment. This is not limited herein.

It should be understood that the steps in the foregoing method embodiments may be completed by using an integrated logic circuit of hardware in the processor or instructions in a form of software. The steps of the method disclosed with reference to embodiments of this application may be directly performed by a hardware processor, or may be performed through a combination of hardware in the processor and a software module.

This application further provides an electronic device. The electronic device may include a PPG assembly, a memory, and a processor. The memory may be configured to store a computer program. The PPG assembly includes n (n≥2) detection modules, where each detection module includes one light source and one photoelectric detector. The processor may be configured to invoke the computer program in the memory, to cause the electronic device to perform the method performed by the electronic device 100 in any one of the foregoing embodiments.

This application further provides an electronic device. The electronic device may include a PPG assembly, a memory, and a processor. The memory may be configured to store a computer program. The PPG assembly includes m (m≥2) groups of detection modules, where each group of detection modules includes one or more detection modules, and each detection module includes one light source and one photoelectric detector. The processor may be configured to invoke the computer program in the memory, to cause the electronic device to perform the method performed by the electronic device 100 in any one of the foregoing embodiments.

This application further provides a chip system. The chip system includes at least one processor, configured to implement functions in the method performed by the electronic device 100 in any one of the foregoing embodiments.

In a possible design, the chip system further includes a memory. The memory is configured to store program instructions and data, and the memory is located inside or outside the processor.

The chip system may include a chip, or may include a chip and another discrete device.

Optionally, there may be one or more processors in the chip system. The processor may be implemented by using hardware, or may be implemented by using software. When the processor is implemented by using the hardware, the processor may be a logic circuit, an integrated circuit, or the like. When the processor is implemented by using the software, the processor may be a general-purpose processor, and is implemented by reading software code stored in the memory.

Optionally, there may be one or more memories in the chip system. The memory may be integrated with the processor, or may be disposed separately from the processor. This is not limited in embodiments of this application. For example, the memory may be a non-transitory processor, for example, a read-only memory ROM. The memory and the processor may be integrated into a same chip, or may be separately disposed on different chips. A type of the memory and a manner of disposing the memory and the processor are not specifically limited in embodiments of this application.

For example, the chip system may be a field programmable gate array (field programmable gate array, FPGA), an application-specific integrated circuit (application-specific integrated circuit, ASIC), a system-on-a-chip (system-on-a-chip, SoC), a central processing unit (central process unit, CPU), a network processor (network processor, NP), a digital signal processor (digital signal processor, DSP), a microcontroller unit (microcontroller unit, MCU), a programmable logic device (programmable logic device, PLD), or another integrated chip.

This application further provides a computer program product. The computer program product includes a computer program (which may also be referred to as code or instructions). When the computer program is run, a computer is caused to perform the method performed by the electronic device 100 in any one of the foregoing embodiments.

This application further provides a computer-readable storage medium. The computer-readable storage medium stores a computer program (which may also be referred to as code or instructions). When the computer program is run, a computer is caused to perform the method performed by the electronic device 100 in any one of the foregoing embodiments.

It should be understood that, the processor in embodiments of this application may be an integrated circuit chip, and has a signal processing capability. In an implementation process, steps in the foregoing method embodiments may be implemented by using an integrated logic circuit of hardware in the processor or instructions in a form of software. The processor may be a general-purpose processor, a digital signal processor (digital signal processor, DSP), an application-specific integrated circuit (application-specific integrated circuit, ASIC), a field programmable gate array (field programmable gate array, FPGA) or another programmable logic device, a discrete gate or a transistor logic device, or a discrete hardware component. It may implement or perform the methods, the steps, and logical block diagrams that are disclosed in embodiments of this application. The general-purpose processor may be a microprocessor, or the processor may be any conventional processor or the like. The steps in the methods disclosed with reference to embodiments of this application may be directly performed by a hardware decoding processor, or may be performed through a combination of hardware in the decoding processor and a software module. The software module may be located in a mature storage medium in the art, for example, a random access memory, a flash memory, a read-only memory, a programmable read-only memory, an electrically erasable programmable memory, or a register. The storage medium is located in the memory, and a processor reads information in the memory and completes the steps in the foregoing methods in combination with hardware of the processor.

In addition, an embodiment of this application further provides an apparatus. The apparatus may be specifically a component or a module, and the apparatus may include one or more processors and memories that are connected to each other. The memory is configured to store a computer program. When the computer program is executed by one or more processors, the apparatus is caused to perform the methods in the foregoing method embodiments.

The apparatus, the computer-readable storage medium, the computer program product, or the chip provided in embodiments of this application is configured to perform the corresponding methods provided above. Therefore, for beneficial effects that can be achieved, refer to beneficial effects in the corresponding methods provided above. Details are not described herein again.

The implementations of this application may be randomly combined to achieve different technical effects.

All or some of the foregoing embodiments may be implemented by using software, hardware, firmware, or any combination thereof. When software is used to implement the technical solutions, all or a part of the technical solutions may be implemented in a form of a computer program product. The computer program product includes one or more computer instructions. When the computer program instructions are loaded and executed on the computer, the procedure or functions according to this application are all or partially generated. The computer may be a general-purpose computer, a dedicated computer, a computer network, or another programmable apparatus. The computer instructions may be stored in a computer-readable storage medium or may be transmitted from a computer-readable storage medium to another computer-readable storage medium. For example, the computer instructions may be transmitted from a website, computer, server, or data center to another website, computer, server, or data center in a wired (for example, a coaxial cable, an optical fiber, or a digital subscriber line) or wireless (for example, infrared, radio, or microwave) manner. The computer-readable storage medium may be any usable medium that can be accessed by the computer, or a data storage device, for example, a server or a data center, integrating one or more usable media. The usable medium may be a magnetic medium (for example, a floppy disk, a hard disk, or a magnetic tape), an optical medium (for example, a DVD), a semiconductor medium (for example, a solid-state drive (solid-state drive, SSD)), or the like.

A person of ordinary skill in the art may understand that all or some of the procedures of the methods in embodiments may be implemented by a computer program instructing relevant hardware. The program may be stored in the computer-readable storage medium. When the program is executed, the procedures of the foregoing method embodiments may be included. The foregoing storage medium includes any medium that can store program code, for example, a ROM, a random access memory RAM, a magnetic disk, or an optical disc.

In summary, what is described above is merely embodiments of the technical solutions of this application, but is not intended to limit the protection scope of this application. Any modification, equivalent replacement, improvement, or the like made based on the disclosure of this application shall fall within the protection scope of this application.

**Claims**

1. A physiological parameter detection method, wherein the method is applied to an electronic device comprising a photoplethysmography PPG assembly, the PPG assembly comprises n (n≥2) detection modules, each detection module comprises one light source and one photoelectric detector, and the method comprises:

collecting n PPG signals via the n detection modules, wherein each PPG signal is obtained in a manner in which a light source in one detection module emits a light ray toward skin and a photoelectric detector in the one detection module detects a light ray reflected from the skin, the n PPG signals comprise at least signals respectively collected by two detection modules with different spacings, and a spacing of a detection module is a distance between a light source and a photoelectric detector in the detection module; and
determining a physiological parameter value of a user based on the n PPG signals, wherein the physiological parameter value is related to an arterial activity of the user.

2. The method according to claim 1, wherein before collecting the n PPG signals via the n detection modules, the method further comprises:

collecting a first PPG signal via one detection module in the n detection modules; and
collecting the n PPG signals via the n detection modules specifically comprises:
when a physiological parameter value reflected by the first PPG signal is abnormal or a pulse reflected by the first PPG signal is incomplete, collecting the n PPG signals via the n detection modules.

3. The method according to claim 2, wherein determining the physiological parameter value based on the n PPG signals specifically comprises:
determining the physiological parameter value based on the n PPG signals and the first PPG signal.

4. The method according to any one of claims 1 to 3, wherein the physiological parameter value is a blood oxygen value, and in any detection module in the PPG assembly, light emitted by a light source comprises light with at least two wavelengths; and
determining the physiological parameter value of the user based on the n PPG signals specifically comprises:

calculating one ratio value R for each PPG signal in the n PPG signals, wherein the ratio value R is for reflecting a ratio between absorption rates of light with different wavelengths in the PPG signal; and
determining the blood oxygen value of the user based on n ratio values R corresponding to the n PPG signals.

5. The method according to claim 4, wherein the blood oxygen value of the user is determined based on the n ratio values R corresponding to the n PPG signals:
inputting the n ratio values R corresponding to the n PPG signals into a first blood oxygen model, to obtain the blood oxygen value of the user, wherein the first blood oxygen model is for representing a mathematical relationship between a plurality of ratio values and blood oxygen, the first blood oxygen model is determined based on a plurality of data sets, and one data set comprises a blood oxygen value of a testee and k ratio values R calculated by using PPG signals collected by k (k≥1) detection modules from the measured person.

6. A physiological parameter detection method, wherein the method is applied to an electronic device comprising a PPG assembly, the PPG assembly comprises m (m≥2) groups of detection modules, each group of detection modules comprises one or more detection modules, each detection module comprises one light source and one photoelectric detector, and the method comprises:

collecting m groups of PPG signals via the m groups of detection modules, wherein each group of PPG signals comprises one PPG signal collected by each detection module in one group of detection modules, one PPG signal is obtained in a manner in which a light source in one detection module emits a light ray toward skin and a photoelectric detector in the one detection module detects a light ray reflected from the skin, the m groups of PPG signals comprise a first group of PPG signals, the first group of PPG signals comprises at least signals respectively collected by two detection modules with different spacings, and a spacing of a detection module is a distance between a light source and a photoelectric detector in the detection module;

determining m physiological parameter values based on the m groups of PPG signals, wherein each physiological parameter value is determined based on one group of PPG signals; and

determining a physiological parameter value of a user based on the m physiological parameter values, wherein the physiological parameter value is related to an arterial activity of the user.

7. The method according to claim 6, wherein two groups of detection modules in the PPG assembly comprise at least a same light source and/or at least a same photoelectric detector.

8. The method according to claim 6 or 7, wherein the physiological parameter value of the user is a largest value, a smallest value, an average value, a median, or a mode of the m physiological parameter values.

9. The method according to any one of claims 6 to 8, wherein the physiological parameter value is a blood oxygen value, and in any detection module in the PPG assembly, light emitted by a light source comprises light with at least two wavelengths; and

one blood oxygen value in the m blood oxygen values is determined based on one or more ratio values R corresponding to one group of PPG signals, one ratio value R is determined based on one PPG signal in one group of PPG signals, and the ratio value R is for reflecting a ratio between absorption rates of light with different wavelengths in the PPG signal.

10. The method according to claim 9, wherein the one blood oxygen value is obtained by inputting the one or more ratio values R into a first blood oxygen model, the first blood oxygen model is for representing a mathematical relationship between a plurality of ratio values and blood oxygen, the first blood oxygen model is determined based on a plurality of data sets, and one data set comprises a blood oxygen value of a testee and k ratio values R calculated by using PPG signals collected by k ($k \geq 1$) detection modules from the measured person.

11. The method according to any one of claims 6 to 10, wherein before collecting the m groups of PPG signals via the m groups of detection modules, the method further comprises:

collecting a first PPG signal via one detection module in the m groups of detection modules; and collecting the m groups of PPG signals via the m groups of detection modules specifically comprises: when a physiological parameter value reflected by the first PPG signal is abnormal or a pulse reflected by the first PPG signal is incomplete, collecting the m groups of PPG signals via the m groups of detection modules.

12. The method according to claim 2 or 11, wherein the method further comprises:
when the physiological parameter value reflected by the first PPG signal is normal or the pulse reflected by the first PPG signal is complete, determining that the physiological parameter value determined based on the first PPG signal is the physiological parameter value of the user.

13. The method according to any one of claims 1 to 12, wherein the physiological parameter value is a value of a first physiological parameter, and the first physiological parameter comprises any one or more of the following: blood oxygen, heart rate, respiratory rate, blood pressure, blood glucose, muscle oxygen, vascular resistance, or brain oxygen.

14. The method according to any one of claims 1 to 13, wherein two detection modules in the PPG assembly comprise at least a same light source or at least a same photoelectric detector.

15. An electronic device, comprising a PPG assembly, a memory, one or more processors, and one or more programs, wherein the PPG assembly comprises n ($n \geq 2$) detection modules, each detection module comprises one light source and one photoelectric detector, and when the one or more processors execute the one or more programs, the electronic device is caused to implement the method according to any one of claims 1 to 5 and 12 to 14.

16. An electronic device, comprising a PPG assembly, a memory, one or more processors, and one or more programs, wherein the PPG assembly comprises m (m≥2) groups of detection modules, each group of detection modules comprises one or more detection modules, each detection module comprises one light source and one photoelectric detector, and when the one or more processors execute the one or more programs, the electronic device is caused to implement the method according to any one of claims 6 to 14.

17. A computer-readable storage medium, comprising instructions, wherein when the instructions are run on an electronic device, the electronic device is caused to perform the method according to any one of claims 1 to 14.

18. A computer program product, wherein when the computer program product is run on a computer, the computer is caused to perform the method according to any one of claims 1 to 14.

LED   PD

Artery
branch

FIG. 1A

FIG. 1B

LED 1    PD    LED 2

Artery
branch

## FIG. 2

Electronic device 100

Wireless communication module
BT/WLAN/GNSS/NFC/IR/FM
[160]

Speaker
[170A]

Receiver
[170B]

Microphone
[170C]

Audio
module
[170]

**Displays 1 to N [194]**

Indicator [192]

Motor [191]

**Processor
[110]**

Sensor module
[180]

Touch sensor
[180A]

**PPG assembly
[193]**

Button [190]

**Internal
memory [121]**

Charging
management
module
[140]

Power
management
module
[141]

Battery [142]

## FIG. 3A

EP 4 767 939 A1

Photoelectric detector

Light source 1

Light source 2

FIG. 3B

| Collect n PPG signals via n detection modules in a PPG assembly, where the n PPG signals include at least signals respectively collected by two detection modules with different spacings, and a spacing of a detection module is a distance between a light source and a photoelectric detector in the detection module | S101 |

| Determine a physiological parameter value of a user based on the n PPG signals, where the physiological parameter value is related to an arterial activity of the user | S102 |

FIG. 4

AC component

Peak

PPG signal

Decompose

Trough

DC component

FIG. 5

Calculate one ratio value R for each PPG signal in n PPG signals, where the ratio value R is for reflecting a ratio between absorption rates of light with different wavelengths in the PPG signal — S201

Determine a blood oxygen value based on n ratio values R corresponding to the n PPG signals — S202

FIG. 6

PD

LED 1

LED 2

FIG. 7

PD

LED 1

LED 2

...

LED n

FIG. 8

FIG. 9

FIG. 10

FIG. 11

FIG. 12

Collect m groups of PPG signals via m groups of detection modules in a PPG assembly, where each group of PPG signals includes one PPG signal collected by each detection module in one group of detection modules — S301

Determine m physiological parameter values based on the m groups of PPG signals, where each physiological parameter value is determined based on one group of PPG signals — S302

Determine a physiological parameter value of a user based on the m physiological parameter values, where the physiological parameter is related to an arterial activity of the user — S303

FIG. 13

FIG. 14

FIG. 15

First group     Second group             m$^{th}$ group

| First group | Second group |  | m$^{th}$ group |
|---|---|---|---|
| LED | LED | | LED |
| PD 1 | PD 1 | | PD 1 |
| PD 2 | PD 2 | ... | PD 2 |
| ... | ... | | ... |
| PD n | PD n | | PD n |

FIG. 16

FIG. 17

FIG. 18

LED 1

Second group

PD 3

PD 1

First group

LED 2

Third group

LED 4

LED 3

PD 2

**FIG. 19**

LED 1

PD 1

PD 2

LED 2

LED 3

PD 3

PD 4

LED 4

LED 5

LED 6

PD 5

PD 6

**FIG. 20**

Collect a first PPG signal via one detection module in a PPG assembly — S401

Determine whether a preset condition is met — S402

Yes

No

S403 — Determine a physiological parameter value determined based on the first PPG signal as a physiological parameter value of a user

Collect a second PPG signal via another detection module in the PPG assembly, where a spacing of the detection module that collects the first PPG signal is different from a spacing of the detection module that collects the second PPG signal — S404

Determine the physiological parameter value of the user based on the first PPG signal and the second PPG signal — S405

FIG. 21

Collect a first PPG signal via one detection module in a PPG assembly — S501

Determine whether a preset condition is met — S502

Yes

No

S503 — Determine a physiological parameter value determined based on the first PPG signal as a physiological parameter value of a user

Determine the physiological parameter value of the user via a plurality of detection modules in the PPG assembly, where the plurality of detection modules include at least detection modules with two spacings — S504

FIG. 22

**Physiological parameter detection apparatus 200**

| | |
|---|---|
| ⌒ 201 | ⌒ 202 |
| Processor | Memory |

⌒ 203

FIG. 23

## INTERNATIONAL SEARCH REPORT

| International application No. |
| --- |
| **PCT/CN2025/093067** |

**A.     CLASSIFICATION OF SUBJECT MATTER**

A61B5/1455(2006.01)i;  A61B5/0205(2006.01)i;  A61B5/00(2006.01)i

According to International Patent Classification (IPC) or to both national classification and IPC

**B.     FIELDS SEARCHED**

Minimum documentation searched (classification system followed by classification symbols)

IPC:  A61B

Documentation searched other than minimum documentation to the extent that such documents are included in the fields searched

Electronic data base consulted during the international search (name of data base and, where practicable, search terms used)

CNABS: CNTXT; ENTXTC; WPABSC; CNKI; VEN; ENTXT; IEEE: 华为, 血氧, 脉搏, 光电容积, 光源, 光电探测器, 距离, 间距, 多通道, 多路, blood oxygen, SpO2, pulse, PPG, light, LED, PD, photodetect+, distance, multi+

**C.     DOCUMENTS CONSIDERED TO BE RELEVANT**

| Category* | Citation of document, with indication, where appropriate, of the relevant passages | Relevant to claim No. |
| --- | --- | --- |
| PX | CN 119867745 A (HUAWEI TECHNOLOGIES CO., LTD.) 25 April 2025 (2025-04-25)<br>claims 1-18 | 1-18 |
| X | CN 115251857 A (BEIJING HONOR DEVICE CO., LTD.) 01 November 2022 (2022-11-01)<br>description, paragraphs [0041]-[0151], and figures 1-13 | 1-5, 12-15, 17, 18 |
| Y | CN 115251857 A (BEIJING HONOR DEVICE CO., LTD.) 01 November 2022 (2022-11-01)<br>description, paragraphs [0041]-[0151], and figures 1-13 | 6-18 |
| Y | CN 117796803 A (GUANGDONG TRANSTEK MEDICAL ELECTRONICS CO., LTD.) 02 April 2024 (2024-04-02)<br>description, paragraphs [0001]-[0109], and figures 1-8 | 6-18 |
| X | TW I827419 B (HON HAI PRECISION INDUSTRY CO., LTD. et al.) 21 December 2023 (2023-12-21)<br>description, paragraphs [0035]-[0107], and figures 1-6 | 1-5, 12-15, 17, 18 |
| Y | TW I827419 B (HON HAI PRECISION INDUSTRY CO., LTD. et al.) 21 December 2023 (2023-12-21)<br>description, paragraphs [0035]-[0107], and figures 1-6 | 6-18 |

☑ Further documents are listed in the continuation of Box C.     ☑ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
| --- | --- |
| **16 July 2025** | **24 July 2025** |

| Name and mailing address of the ISA/CN | Authorized officer |
| --- | --- |
| **China National Intellectual Property Administration (ISA/CN)**<br>**China No. 6, Xitucheng Road, Jimenqiao, Haidian District, Beijing 100088** | |
| | Telephone No. |

Form PCT/ISA/210 (second sheet) (July 2022)

**EP 4 767 939 A1**

**INTERNATIONAL SEARCH REPORT**

| International application No. |
| --- |
| **PCT/CN2025/093067** |

**C.    DOCUMENTS CONSIDERED TO BE RELEVANT**

| Category* | Citation of document, with indication, where appropriate, of the relevant passages | Relevant to claim No. |
| --- | --- | --- |
| X | US 2017343417 A1 (SONY MOBILE COMMUNICATIONS INC.) 30 November 2017 (2017-11-30) description, paragraphs [0021]-[0193], and figures 1-5 | 1, 2, 12-15, 17, 18 |
| Y | US 2017343417 A1 (SONY MOBILE COMMUNICATIONS INC.) 30 November 2017 (2017-11-30) description, paragraphs [0021]-[0193], and figures 1-5 | 6-18 |
| X | US 2021330207 A1 (FITBIT INC.) 28 October 2021 (2021-10-28) description, paragraphs [0027]-[0154], and figures 1A-14 | 1, 2, 12-15, 17, 18 |
| Y | US 2021330207 A1 (FITBIT INC.) 28 October 2021 (2021-10-28) description, paragraphs [0027]-[0154], and figures 1A-14 | 6-18 |
| A | CN 112741626 A (HUAWEI TECHNOLOGIES CO., LTD.) 04 May 2021 (2021-05-04) entire document | 1-18 |
| A | CN 219895716 U (CHIPSEA TECHNOLOGY (SHENZHEN) CO., LTD.) 27 October 2023 (2023-10-27) entire document | 1-18 |

Form PCT/ISA/210 (second sheet) (July 2022)

**INTERNATIONAL SEARCH REPORT**
Information on patent family members

| International application No. |
|---|
| **PCT/CN2025/093067** |

| Patent document<br>cited in search report | | | Publication date<br>(day/month/year) | Patent family member(s) | | | Publication date<br>(day/month/year) |
|---|---|---|---|---|---|---|---|
| CN | 119867745 | A | 25 April 2025 | None | | | |
| CN | 115251857 | A | 01 November 2022 | None | | | |
| CN | 117796803 | A | 02 April 2024 | None | | | |
| TW | I827419 | B | 21 December 2023 | TW | 202421060 | A | 01 June 2024 |
| US | 2017343417 | A1 | 30 November 2017 | US | 10274373 | B2 | 30 April 2019 |
| US | 2021330207 | A1 | 28 October 2021 | None | | | |
| CN | 112741626 | A | 04 May 2021 | None | | | |
| CN | 219895716 | U | 27 October 2023 | None | | | |

Form PCT/ISA/210 (patent family annex) (July 2022)

**EP 4 767 939 A1**